# EUROPÄISCHE PATENTSCHRIFT

(11) **EP 2 984 150 B1**
(45) Veröffentlichungstag und Bekanntmachung des Hinweises auf die Patenterteilung: **28.12.2016**
(21) Anmeldenummer: 14709196.1
(22) Anmeldetag: 10.03.2014
(51) Int. Cl.: C09K 11/06, C07C 211/61, H01L 51/50

(54) **MATERIALIEN FÜR ELEKTRONISCHE VORRICHTUNGEN**
MATERIALS FOR ELECTRONIC DEVICES
MATÉRIAUX POUR DISPOSITIFS ÉLECTRONIQUES

(30) Priorität: 08.04.2013 EP 13001798
(43) Veröffentlichungstag der Anmeldung: 17.02.2016
(73) Patentinhaber: Merck Patent GmbH, 64293 Darmstadt (DE)
(72) Erfinder: HEIL, Holger, 60389 Frankfurt am Main (DE); GERHARD, Anja, 63329 Egelsbach (DE); ECKES, Fabrice, 68300 Saint Louis (FR); DARSY, Amandine, 60318 Frankfurt am Main (DE)
(86) Internationale Anmeldenummer: PCT/EP2014/000613
(87) Internationale Veröffentlichungsnummer: WO 2014/166571

(56) Entgegenhaltungen:
- US-A1- 2007 009 758
- US-A1- 2007 252 511
- US-A1- 2007 292 714

## Beschreibung

Die vorliegende Erfindung betrifft eine Verbindung einer Formel (I). Die Verbindung stellt ein Pyren dar, das mindestens zwei Arylaminogruppen aufweist, welche über eine verbrückende Gruppe miteinander verbunden sind. Die Verbindung eignet sich zur Verwendung als Funktionsmaterial in einer elektronischen Vorrichtung, insbesondere einer organischen Elektrolumineszenzvorrichtung (OLED).

Unter dem Begriff elektronische Vorrichtung werden gemäß der vorliegenden Anmeldung allgemein elektronische Vorrichtungen verstanden, welche organische Materialien enthalten. Bevorzugt werden darunter OLEDs verstanden sowie weitere Ausführungsformen von elektronischen Vorrichtungen enthaltend organische Materialien, die an späterer Stelle offenbart werden.

Allgemein wird unter der Bezeichnung OLED eine elektronische Vorrichtung verstanden, welche mindestens ein organisches Material enthält und unter Anlegen einer elektrischen Spannung Licht emittiert. Der genaue Aufbau von OLEDs ist unter anderem in US 4539507, US 5151629, EP 0676461 und WO 98/27136 beschrieben.

Betreffend die Leistungsdaten der elektronischen Vorrichtungen sind weitere Verbesserungen erforderlich, insbesondere in Hinblick auf eine breite kommerzielle Verwendung, beispielsweise in Displays oder als Lichtquellen. Von besonderer Bedeutung sind in diesem Zusammenhang die Lebensdauer, die Effizienz und die Betriebsspannung der elektronischen Vorrichtungen sowie die realisierten Farbwerte. Insbesondere bei blau emittierenden OLEDs besteht Verbesserungspotential bezüglich der Lebensdauer der Vorrichtungen und den erreichten Farbwerten des emittierten Lichts.

Ein wichtiger Ansatzpunkt, um die genannten Verbesserungen zu erreichen, ist die Auswahl der emittierenden Verbindung, die in der elektronischen Vorrichtung eingesetzt wird.

Im Stand der Technik sind als blau fluoreszierende Emitter eine Vielzahl an Verbindungen bekannt, insbesondere Arylamine mit einer oder mehreren kondensierten Arylgruppen.

Beispielsweise sind in EP 1604974 und US 2011/0156011 Diarylamino-Pyren-Verbindungen offenbart, welche sich als blau emittierende Verbindungen in elektronischen Vorrichtungen eignen. Die Verbindungen weisen eine zentrale Pyrengruppe auf, an die eine, zwei oder mehr Diarylaminogruppen binden. Weiterhin werden in US 2012/0032152 Diarylamino-Pyren-Verbindungen offenbart, welche sich als blau emittierende Verbindungen in elektronischen Vorrichtungen eignen. Die Verbindungen weisen eine Pyrengruppe auf, an die eine Arylgruppe bindet, an die ihrerseits eine Diarylaminogruppe bindet. Als zweiter Substituent ist an die Pyrengruppe eine Diarylaminogruppe gebunden.

Es besteht jedoch allgemein Bedarf an alternativen Funktionsmaterialien, die zur Verwendung in elektronischen Vorrichtungen, insbesondere OLEDs, geeignet sind. Auch sind die technischen Erfordernisse an die Materialien, insbesondere in der Funktion als blau emittierende Verbindungen, noch nicht vollständig zufriedenstellend erfüllt. Eine spezielle technische Aufgabe kann daher in der Verbesserung der Leistungsdaten der elektronischen Vorrichtung liegen, zu denen insbesondere Lebensdauer, tiefblaue Farbkoordinaten des emittierten Lichts und/oder Leistungseffizienz zählen.

Es besteht Bedarf an Funktionsmaterialien für OLEDs, welche bei Verwendung als Emitter tiefblaue Farbkoordinaten aufweisen, und welche eine schmale Emissionsbande aufweisen. Letzteres ist besonders bei Kombination von mehreren Emittermaterialien mit unterschiedlicher Lage ihrer Emissionsbanden von Bedeutung, deren emittiertes Licht gemischt wird, beispielsweise zu weißem Licht.

Auch ist es von Interesse, die Temperaturstabilität der Verbindungen zur Verwendung in elektronischen Vorrichtungen zu erhöhen, zum einen aufgrund der erhöhten Temperatur der Vorrichtungen im Betrieb, zum anderen aufgrund der sehr hohen Temperaturen, die bei der Reinigung der Materialien durch Sublimation und bei der der Aufbringung der Materialien durch Gasphasen-Abscheidung im Herstellungsprozess der elektronischen Vorrichtung auftreten.

Zusammenfassend liegt also die technische Aufgabe vor, alternative Funktionsmaterialien zur Verwendung in elektronischen Vorrichtungen bereitzustellen. Insbesondere liegt die Aufgabe vor, Materialien bereitzustellen, welche verbesserte Eigenschaften aufweisen und/oder verbesserte Eigenschaften der elektronischen Vorrichtung enthaltend das Material bewirken, insbesondere in den oben genannten Punkten.

Trotz der Vielfalt an Pyren-Arylamin-Verbindungen, die bis heute zur Verwendung in elektronischen Vorrichtungen vorgeschlagen wurden, wurde im Stand der Technik bis jetzt in keinem Fall gelehrt, die Arylaminogruppen, welche an den zentralen Pyren-Grundkörper binden, über eine verbrückende Gruppe miteinander zu verbinden.

Überraschend wurde nun gefunden, dass Pyren-Verbindungen, welche mindestens zwei Diarylaminogruppen aufweisen, wobei die Diarylaminogruppen über eine verbrückende Gruppe miteinander verbunden sind, hervorragend zur Verwendung als Funktionsmaterialien in elektronischen Vorrichtungen geeignet sind und damit die oben genannte technische Aufgabe lösen.

Bevorzugt weisen sie tiefblaue Farbkoordinaten, eine geringe Breite der Emissionsbande, und/oder eine hohe Temperaturstabilität auf, und/oder führen bei Verwendung in einer elektronischen Vorrichtung zu vorteilhaften Leistungsdaten.

Gegenstand der Erfindung ist somit eine Verbindung einer Formel (I) 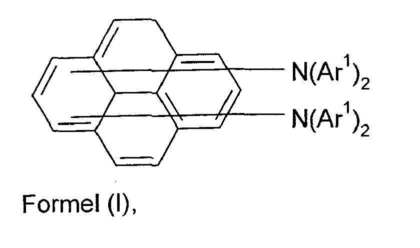 wobei die Pyrengruppe an jeder freien Position mit einem Rest R¹ substituiert sein kann, und wobei gilt:
in der Formel (I) ist mindestens eine Gruppe Ar¹, die Bestandteil einer Gruppe N(Ar¹)₂ ist, über eine Gruppe X mit einer anderen Gruppe Ar¹, die Bestandteil einer anderen Gruppe N(Ar¹)₂ ist, verknüpft;
   - Ar¹: ist bei jedem Auftreten gleich oder verschieden ein aromatisches oder heteroaromatisches Ringsystem mit 6 bis 30 aromatischen Ringatomen, das mit einem oder mehreren Resten R³ substituiert sein kann, wobei Gruppen Ar¹ dabei über Gruppen X verknüpft sein können;
   - X: ist bei jedem Auftreten gleich oder verschieden eine Einfachbindung, oder eine divalente Gruppe gewählt aus einer Aryl- oder Heteroarylgruppe mit 6 bis 20 aromatischen Ringatomen, die mit einem oder mehreren Resten R² substituiert sein kann, BR², C(R²)₂, -R²C=CR²-, C(=O), Si(R²)₂, NR², PR², P(=O)R², O, S, S(=O), S(=O)₂, oder eine Kombination aus 2,3,4 oder 5 gleichen oder verschiedenen der genannten divalenten Gruppen;
   - R¹: ist bei jedem Auftreten gleich oder verschieden H, D, F, C(=O)R⁴, CN, Si(R⁴₎₃, N(Ar¹)₂, N(R⁴)₂, P(=O)(R⁴)₂, OAr¹, S(=O)R⁴, S(=O)₂R⁴, eine geradkettige Alkyl- oder Alkoxygruppe mit 1 bis 20 C-Atomen oder eine verzweigte oder cyclische Alkyl- oder Alkoxygruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁴ substituiert sein können und wobei eine oder mehrere CH₂-Gruppen in den oben genannten Gruppen durch -R⁴C=CR⁴-, -C=C-, Si(R⁴)₂, C=O, C=NR⁴, -C(=O)O-, -C(=O)NR⁴-, NR⁴, P(=O)(R⁴), -O-, -S-, SO oder SO₂ ersetzt sein können, oder ein aromatisches oder heteroaromatisches Ringsystem mit 5 bis 30 aromatischen Ringatomen, das jeweils durch einen oder mehrere Reste R⁴ substituiert sein kann, wobei zwei oder mehr Reste R¹ miteinander verknüpft sein können und einen Ring bilden können;
   - R²: ist bei jedem Auftreten gleich oder verschieden H, D, F, C(=O)R⁴, CN, Si(R⁴)₃, N(R⁴)₂, P(=O)(R⁴)₂, S(=O)R₄, S(=O)₂R⁴, eine geradkettige Alkyl- oder Alkoxygruppe mit 1 bis 20 C-Atomen oder eine verzweigte oder cyclische Alkyl- oder Alkoxygruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁴ substituiert sein können und wobei eine oder mehrere CH₂-Gruppen in den oben genannten Gruppen durch -R⁴C=CR⁴-, -C≡C-, Si(R⁴)₂, C=O, C=NR⁴, -C(=O)O-, -C(=O)NR⁴-, NR⁴, P(=O)(R⁴), -O-, -S-, SO oder SO₂ ersetzt sein können, oder ein aromatisches oder heteroaromatisches Ringsystem mit 5 bis 30 aromatischen Ringatomen, das jeweils durch einen oder mehrere Reste R⁴ substituiert sein kann, wobei zwei oder mehr Reste R² miteinander verknüpft sein können und einen Ring bilden können;
   - R³: ist bei jedem Auftreten gleich oder verschieden H, D, F, C(=O)R⁴ CN, Si(R⁴)₃, N(R⁴)₂, P(=O)(R⁴)₂, S(=O)R ⁴ S(=O)₂R⁴, eine geradkettige Alkyl- oder Alkoxygruppe mit 1 bis 20 C-Atomen oder eine verzweigte oder cyclische Alkyl- oder Alkoxygruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁴ substituiert sein können und wobei eine oder mehrere CH₂-Gruppen in den oben genannten Gruppen durch -R⁴C=CR⁴-, -C=C-, Si(R⁴)₂, C=O, C=NR⁴, -C(=O)O-, -C(=O)NR⁴-, NR⁴, P(=O)(R⁴), -O-, -S-, SO oder SO₂ ersetzt sein können, oder ein aromatisches oder heteroaromatisches Ringsystem mit 5 bis 30 aromatischen Ringatomen, das jeweils durch einen oder mehrere Reste R⁴ substituiert sein kann, wobei zwei oder mehr Reste R³ miteinander verknüpft sein können und einen Ring bilden können;
   - R⁴: ist bei jedem Auftreten gleich oder verschieden H, D, F oder ein aliphatischer, aromatischer oder heteroaromatischer organischer Rest mit 1 bis 20 C-Atomen, in dem auch ein oder mehrere H-Atome durch D oder F ersetzt sein können; dabei können zwei oder mehr Substituenten R⁴ miteinander verknüpft sein und einen Ring bilden.

Die Darstellung der Bindung der Gruppen N(Ar₁)₂ in Formel (I) an das Pyren bedeutet, dass die Bindung an jeder beliebigen freien Position des Pyrens erfolgen kann.

Es wird die folgende Nummerierung des Pyrens verwendet:

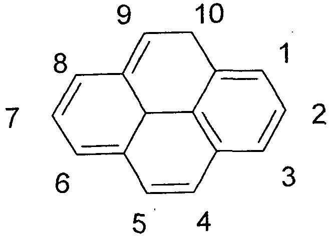

Eine Arylgruppe im Sinne dieser Erfindung enthält 6 bis 60 aromatische Ringatome; eine Heteroarylgruppe im Sinne dieser Erfindung enthält 5 bis 60 aromatische Ringatome, von denen mindestens eines ein Heteroatom darstellt. Die Heteroatome sind bevorzugt ausgewählt aus N, O und S. Dies stellt die grundlegende Definition dar. Werden in der Beschreibung der vorliegenden Erfindung andere Bevorzugungen angegeben, beispielsweise bezüglich der Zahl der aromatischen Ringatome oder der enthaltenen Heteroatome, so gelten diese.

Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin oder Thiophen, oder ein kondensierter (annellierter) aromatischer bzw. heteroaromatischer Polycyclus, beispielsweise Naphthalin, Phenanthren, Chinolin oder Carbazol verstanden. Ein kondensierter (annellierter) aromatischer bzw. heteroaromatischer Polycyclus besteht im Sinne der vorliegenden Anmeldung aus zwei oder mehr miteinander kondensierten einfachen aromatischen bzw. heteroaromatischen Cyclen.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazof, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Ein aromatisches Ringsystem im Sinne dieser Erfindung enthält 6 bis 60 C-Atome im Ringsystem. Ein heteroaromatisches Ringsystem im Sinne dieser Erfindung enthält 5 bis 60 aromatische Ringatome, von denen mindestens eines ein Heteroatom darstellt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein sp³-hybridisiertes C-, Si-, N- oder O-Atom, ein sp²-hybridisiertes C- oder N-Atom oder ein sp-hybridisiertes C-Atom, verbunden sein können. So sollen beispielsweise auch Systeme wie 9,9'-Spirobifluoren, 9,9'-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkyl-, Alkenyl- oder Alkinylgruppe oder durch eine Silylgruppe verbunden sind. Weiterhin werden auch Systeme, in denen zwei oder mehr Aryl- oder Heteroarylgruppen über Einfachbindungen miteinander verknüpft sind, als aromatische oder heteroaromatische Ringsysteme im Sinne dieser Erfindung verstanden, wie beispielsweise Systeme wie Biphenyl, Terphenyl oder Diphenyltriazin.

Unter einem aromatischen oder heteroaromatischen Ringsystem mit 5-60 aromatischen Ringatomen, welches noch jeweils mit Resten wie oben definiert substituiert sein kann und welches über beliebige Positionen am Aromaten bzw. Heteroarömaten verknüpft sein kann, werden insbesondere Gruppen verstanden, die abgeleitet sind von Benzol, Naphthalin, Anthracen, Benzanthracen, Phenanthren, Benzphenanthren, Pyren, Chrysen, Perylen, Fluoranthen, Naphthacen, Pentacen, Benzpyren, Biphenyl, Biphenylen, Terphenyl, Terphenylen, Quaterphenyl, Fluoren, Spirobifluoren, Dihydrophenanthren, Dihydropyren, Tetrahydropyren, cis- oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, lndazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Kombinationen dieser Gruppen.

Im Rahmen der vorliegenden Erfindung werden unter einer geradkettigen Alkylgruppe mit 1 bis 40 C-Atomen bzw. einer verzweigten oder cyclischen Alkylgruppe mit 3 bis 40 C-Atomen bzw. einer Alkenyl- oder Alkinylgruppe mit 2 bis 40 C-Atomen, in der auch einzelne H-Atome oder CH₂-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neoPentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer Alkoxy- oder Thioalkylgruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexyloxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Unter der Formulierung, dass zwei oder mehr Reste miteinander einen Ring bilden können, soll im Rahmen der vorliegenden Anmeldung unter anderem verstanden werden, dass die beiden Reste miteinander durch eine chemische Bindung verknüpft sind. Dies wird durch das folgende Schema verdeutlicht:

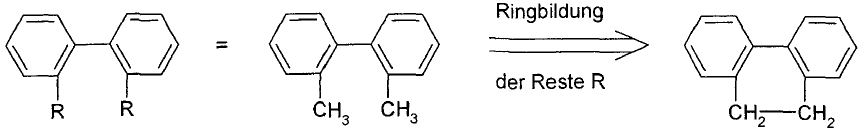

Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

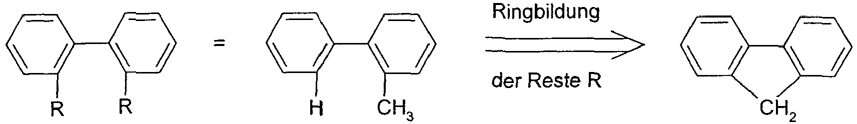

In einer bevorzugten Ausführungsform ist die Gruppe X bei jedem Auftreten gleich oder verschieden eine Einfachbindung oder eine divalente Gruppe gewählt aus einer Aryl- oder Heteroarylgruppe mit 6 bis 14 aromatischen Ringatomen, die mit einem oder mehreren Resten R² substituiert sein kann, C(R²₎₂, C(=O), Si(R²)₂, NR², O oder S, oder eine Kombination aus 2, 3 oder 4 gleichen oder verschiedenen dieser divalenten Gruppen.

Besonders bevorzugt ist X bei jedem Auftreten gleich oder verschieden eine Einfachbindung oder eine divalente Gruppe gewählt aus einer Aryl- oder Heteroarylgruppe mit 6 aromatischen Ringatomen, die mit einem oder mehreren Resten R² substituiert sein kann, C(R²)₂, C(=O), Si(R²)₂, NR², O, S, -C(R²)₂-C(R²)₂-, oder -NR¹- (Ar²)ₙ- NR¹-, wobei n gleich 1 oder 2 ist, und Ar² eine Aryl- oder Heteroarylgruppe mit 6 aromatischen Ringatomen ist, die mit einem oder mehreren Resten R² substituiert sein kann.

Ganz besonders bevorzugt ist X bei jedem Auftreten gleich oder verschieden eine divalente Gruppe gewählt aus C(R²)₂, NR², O und S, nochmals stärker bevorzugt eine divalente Gruppe gewählt aus C(R²)₂ und NR²_{.}

In einer bevorzugten Ausführungsform der Erfindung bilden Gruppen R², die an dasselbe Atom einer Einheit X binden, miteinander einen Ring, so dass ein Spiro-Atom entsteht. Bevorzugt ist dies ein Fünfring oder ein Sechsring, wobei die Gruppen R² bevorzugt Arylgruppen oder Alkylgruppen sind. Ganz besonders bevorzugt ist die Gruppe X in diesen Fällen gewählt aus Gruppen der folgenden Formeln (X-1) bis (X-10)

| | | |
|---|---|---|
| 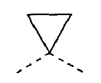 | 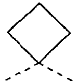 | 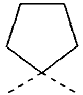 |
| Formel (X-1 ) | Formel (X-2) | Formel (X-3) |
| 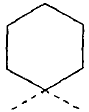 | 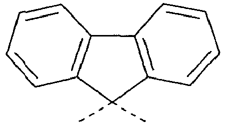 | 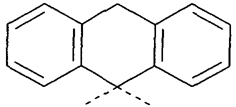 |
| Formel (X-4) | Formel (X-5) | Formel (X-6) |
| 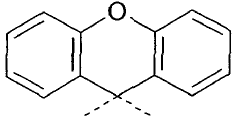 | 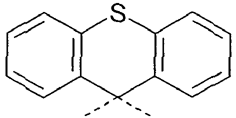 | 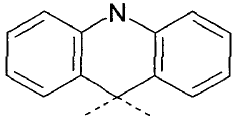 |
| Formel (X-7) | Formel (X-8) | Formel (X-9) |
| 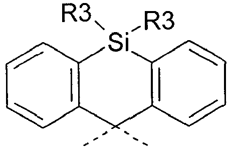 | | |
| Formel (X-10) | | |

wobei die abgebildeten Gruppen in den unsubstituiert dargestellten Positionen wahlweise mit einem oder mehreren Resten R⁴ substituiert sein können, und wobei die gestrichelten Linien Bindungen an den Rest der Verbindung darstellen.

Es ist bevorzugt, dass in der Verbindung der Formel (I) genau eine oder genau 2 Gruppen X vorliegen. Entsprechend sind bevorzugt genau 1 Paar von Gruppen Ar¹ oder genau 2 Paare von Gruppen Ar¹ miteinander über jeweils eine Gruppe X verknüpft.

In einer bevorzugten Ausführungsform ist Ar¹ bei jedem Auftreten gleich oder verschieden ein aromatisches oder heteroaromatisches Ringsystem mit 6 bis 18 aromatischen Ringatomen, das mit einem oder mehreren Resten R³ substituiert sein kann. Besonders bevorzugt ist Ar¹ bei jedem Auftreten gleich oder verschieden ein aromatisches Ringsystem mit 6 bis 12 aromatischen Ringatomen, das mit einem oder mehreren Resten R³ substituiert sein kann. Ganz besonders bevorzugt ist Ar¹ Phenyl, das mit einem oder mehreren Resten R³ substituiert sein kann.

Zwei an dasselbe Stickstoffatom bindende Gruppen Ar¹ können jeweils über eine Einfachbindung verbunden sein. Bevorzugt wird dabei ein Carbazol gebildet.

Bevorzugt ist R¹ bei jedem Auftreten gleich oder verschieden H, D, F, CN, Si(R⁴)₃, N(Ar¹)₂, eine geradkettige Alkyl- oder Alkoxygruppe mit 1 bis 20 C-Atomen oder eine verzweigte oder cyclische Alkyl- oder Alkoxygruppe mit 3 bis 20 C-Atomen, wobei die oben genannten Gruppen jeweils mit einem oder mehreren Resten R⁴ substituiert sein können und wobei in den oben genannten Gruppen eine oder mehrere CH₂-Gruppen durch -C≡C-, -R⁴C=CR⁴₋, Si(R⁴)₂, C=O, C=NR⁴, -NR⁴-, -O-, -S-, -C(=O)O- oder -C(=O)NR⁴- ersetzt sein können, oder ein aromatisches oder heteroaromatisches Ringsystem mit 5 bis 20 aromatischen Ringatomen, das jeweils mit einem oder mehreren Resten R⁴ substituiert sein kann, wobei zwei oder mehr Reste R¹ miteinander verknüpft sein können und einen Ring bilden können.

Besonders bevorzugt ist die Pyrengruppe mit mindestens einem Rest R¹ substituiert, der nicht gleich H ist. Ganz besonders bevorzugt ist die Pyrengruppe mit mindestens einem R¹ substituiert, das gewählt ist aus einer geradkettigen Alkylgruppe mit 1 bis 8 C-Atomen oder einer verzweigten oder cyclischen Alkylgruppe mit 3 bis 8 C-Atomen, wobei die Alkylgruppen mit einem oder mehreren Resten R⁴ substituiert sein können, oder aus einer Aryl- oder Heteroarylgruppe mit 6 bis 14 aromatischen Ringatomen, die mit einem oder mehreren Resten R⁴ substituiert sein kann.

Es ist bevorzugt, dass in dem Fall, dass die Gruppen N(Ar¹)₂ in Positionen gewählt aus Positionen 6, 7 und 8 gebunden sind, die Verbindung in Position 2 des Pyrengrundkörpers eine Gruppe R¹ aufweist, die nicht gleich H ist, und die bevorzugt gewählt ist aus einer geradkettigen Alkylgruppe mit 1 bis 8 C-Atomen oder einer verzweigten oder cyclischen Alkylgruppe mit 3 bis 8 C-Atomen, wobei die Alkylgruppen mit einem oder mehreren Resten R⁴ substituiert sein können, oder aus einer Aryl- oder Heteroarylgruppe mit 6 bis 14 aromatischen Ringatomen, die mit einem oder mehreren Resten R⁴ substituiert sein kann.

Es ist bevorzugt, dass in dem Fall, dass die Gruppen N(Ar¹)₂ in Positionen gewählt aus Positionen 1, 2 und 3 gebunden sind, die Verbindung in Position 7 des Pyrengrundkörpers eine Gruppe R¹ aufweist, die nicht gleich H ist, und die bevorzugt gewählt ist aus einer geradkettigen Alkylgruppe mit 1 bis 8 C-Atomen oder einer verzweigten oder cyclischen Alkylgruppe mit 3 bis 8 C-Atomen, wobei die Alkylgruppen mit einem oder mehreren Resten R⁴ substituiert sein können, oder aus einer Aryl- oder Heteroarylgruppe mit 6 bis 14 aromatischen Ringatomen, die mit einem oder mehreren Resten R⁴ substituiert sein kann.

Bevorzugt ist R² bei jedem Auftreten gleich oder verschieden F, CN, eine geradkettige Alkylgruppe mit 1 bis 20 C-Atomen oder eine verzweigte oder cyclische Alkylgruppe mit 3 bis 20 C-Atomen, wobei die oben genannten Gruppen jeweils mit einem oder mehreren Resten R⁴ substituiert sein können und wobei in den oben genannten Gruppen eine oder mehrere CH₂-Gruppen durch -C=C-, -R⁴C=CR⁴-, Si(R⁴)₂, C=O, C=NR⁴, -NR⁴-, -O-, -S-, -C(=O)O- oder -C(=O)NR⁴- ersetzt sein können, oder ein aromatisches oder heteroaromatisches Ringsystem mit 5 bis 20 aromatischen Ringatomen, das jeweils mit einem oder mehreren Resten R⁴ substituiert sein kann, wobei zwei oder mehr Reste R² miteinander verknüpft sein können und einen Ring bilden können.

Besonders bevorzugt sind Reste R² an einer Gruppe X, die C(R²)₂ oder Si(R²)₂ darstellt, gewählt aus einer geradkettigen Alkylgruppe mit 1 bis 8 C-Atomen oder einer verzweigten oder cyclischen Alkylgruppe mit 3 bis 8 C-Atomen, wobei die Alkylgruppen mit einem oder mehreren Resten R⁴ substituiert sein können, oder aus einer Aryl- oder Heteroarylgruppe mit 6 bis 14 aromatischen Ringatomen, die mit einem oder mehreren Resten R⁴ substituiert sein kann. Bevorzugt sind solche Gruppen R² miteinander verknüpft, besonders bevorzugt derart, dass eine Gruppe nach einer der Formeln (X-1) bis (X-10) entsteht.

Besonders bevorzugt sind Reste R² an einer Gruppe X, die NR² oder PR² oder P(=O)R² darstellt, gewählt aus Aryl- oder Heteroarylgruppen mit 6 bis 14 aromatischen Ringatomen, die mit einem oder mehreren Resten R⁴ substituiert sein können.

Bevorzugt ist R³ bei jedem Auftreten gleich oder verschieden gewählt aus H, D, F, CN, Si(R⁴)₃, einer geradkettigen Alkyl- oder Alkoxygruppe mit 1 bis 20 C-Atomen oder einer verzweigten oder cyclischen Alkyl- oder Alkoxygruppe mit 3 bis 20 C-Atomen, wobei die oben genannten Gruppen jeweils mit einem oder mehreren Resten R⁴ substituiert sein können und wobei in den oben genannten Gruppen eine oder mehrere CH₂-Gruppen durch - C≡C-, -R⁴C=CR⁴-, Si(R⁴)₂, C=O, C=NR⁴, -NR⁴-, -O-, -S-, -C(=O)O- oder -C(=O)NR⁴- ersetzt sein können, oder einem aromatischen oder heteroaromatischen Ringsystem mit 5 bis 20 aromatischen Ringatomen, das jeweils mit einem oder mehreren Resten R⁴ substituiert sein kann, wobei zwei oder mehr Reste R³ miteinander verknüpft sein können und einen Ring bilden können.

Bevorzugt sind die Gruppen N(Ar¹)₂ in Formel (I) an Positionen gewählt aus den Positionen 1, 3, 6 und 8 des Pyrens gebunden. Besonders bevorzugt ist es dabei, dass miteinander über Gruppen X verbundene Gruppe N(Ar¹)₂ an Positionen 1 und 3 des Pyrens gebunden sind.

Bevorzugt sind die folgenden Ausführungsformen von Verbindungen der Formel (I), entsprechend Formeln (I-1) bis (I-5): 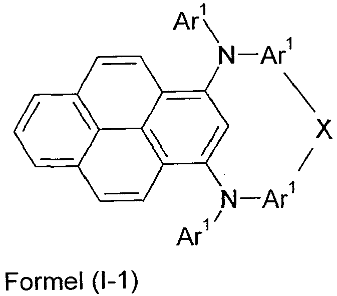 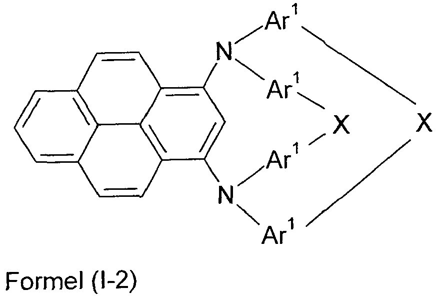 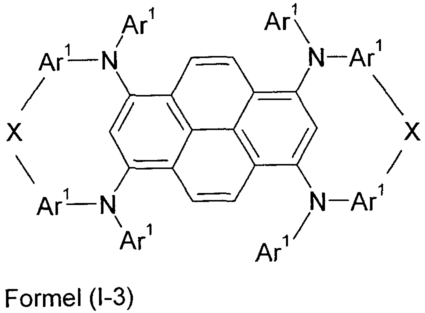 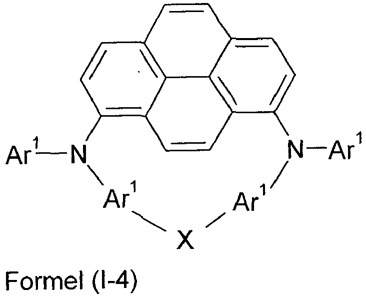 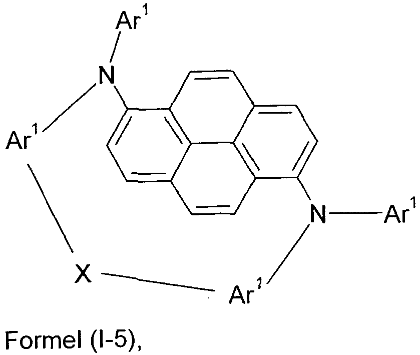 wobei die Pyrengruppe an jeder freien Position mit einem Rest R¹ substituiert sein kann, und wobei die Gruppen definiert sind wie oben.

Es ist bevorzugt, dass die Verbindung der Formel (I) gewählt ist aus Verbindungen nach einer der Formeln (I-1) bis (I-5), wobei X gewählt ist aus den oben angegebenen bevorzugten Ausführungsformen, Ar¹ gewählt ist aus den oben angegebenen bevorzugten Ausführungsformen, R¹ gewählt ist aus den oben angegebenen Ausführungsformen, R² gewählt ist aus den oben angegebenen bevorzugten Ausführungsformen, und R³ gewählt ist aus den oben angegebenen bevorzugten Ausführungsformen.

Besonders bevorzugt entspricht die erfindungsgemäße Verbindung der Formel (I-1).

Es ist weiterhin bevorzugt, dass die Verbindung der Formel (I-1) entspricht, und
- X: ist gewählt aus C(R²)₂, NR², O und S; und
- Ar¹: ist bei jedem Auftreten gleich oder verschieden gewählt aus einem aromatischen Ringsystem mit 6 bis 12 aromatischen Ringatomen, bevorzugt einer Phenylgruppe, wobei das aromatische Ringsystem mit einem oder mehreren Resten R³ substituiert sein kann; und
- R¹: ist bei jedem Auftreten gleich oder verschieden H, D, F, CN, Si(R⁴)₃, N(Ar¹)₂, eine geradkettige Alkyl- oder Alkoxygruppe mit 1 bis 20 C-Atomen oder eine verzweigte oder cyclische Alkyl- oder Alkoxygruppe mit 3 bis 20 C-Atomen, wobei die oben genannten Gruppen jeweils mit einem oder mehreren Resten R⁴ substituiert sein können und wobei in den oben genannten Gruppen eine oder mehrere CH₂-Gruppen durch -C=C-, -R⁴C=CR⁴-, Si(R⁴)₂, C=O, C=NR⁴, -NR⁴-, -O-, -S-, -C(=O)O- oder -C(=O)NR⁴- ersetzt sein können, oder ein aromatisches oder heteroaromatisches Ringsystem mit 5 bis 20 aromatischen Ringatomen, das jeweils mit einem oder mehreren Resten R⁴ substituiert sein kann, wobei zwei oder mehr Reste R¹ miteinander verknüpft sein können und einen Ring bilden können; und
- R²: ist bei jedem Auftreten gleich oder verschieden F, CN, eine geradkettige Alkylgruppe mit 1 bis 20 C-Atomen oder eine verzweigte oder cyclische Alkylgruppe mit 3 bis 20 C-Atomen, wobei die oben genannten Gruppen jeweils mit einem oder mehreren Resten R⁴ substituiert sein können und wobei in den oben genannten Gruppen eine oder mehrere CH₂-Gruppen durch - C=C-, -R⁴C=CR⁴-, Si(R⁴)₂, C=O, C=NR⁴, -NR⁴-, -O-, -S-, -C(=O)O- oder -C(=O)NR⁴- ersetzt sein können, oder ein aromatisches oder heteroaromatisches Ringsystem mit 5 bis 20 aromatischen Ringatomen, das jeweils mit einem oder mehreren Resten R⁴ substituiert sein kann, wobei zwei oder mehr Reste R² miteinander verknüpft sein können und einen Ring bilden können; und

- R³: ist bei jedem Auftreten gleich oder verschieden H, D, F, CN, Si(R⁴)₃, eine geradkettige Alkyl- oder Alkoxygruppe mit 1 bis 20 C-Atomen oder eine verzweigte oder cyclische Alkyl- oder Alkoxygruppe mit 3 bis 20 C-Atomen, wobei die oben genannten Gruppen jeweils mit einem oder mehreren Resten R⁴ substituiert sein können und wobei in den oben genannten Gruppen eine oder mehrere CH₂-Gruppen durch -C=C-, -R⁴C=CR⁴-, Si(R⁴)₂, C=O, C=NR⁴, -NR⁴-, -O-, -S-, -C(=O)O- oder -C(=O)NR⁴- ersetzt sein können, oder ein aromatisches oder heteroaromatisches Ringsystem mit 5 bis 20 aromatischen Ringatomen, das jeweils mit einem oder mehreren Resten R⁴ substituiert sein kann, wobei zwei oder mehr Reste R³ miteinander verknüpft sein können und einen Ring bilden können; und
- R⁴: ist definiert wie oben.

Wenn Ar¹ eine wahlweise mit Resten R³ substituierte Phenylgruppe ist, ist es allgemein bevorzugt, dass die Bindung an die Gruppe X und an das Stickstoffatom an der Phenylgruppe in meta-Position zueinander stehen.

Weiterhin ist es allgemein bevorzugt, bevorzugte Ausführungsformen der Formel (I) sowie der Gruppen der Formel (I) miteinander zu kombinieren.

Beispiele für erfindungsgemäße Verbindungen sind in der folgenden Tabelle aufgeführt.

| | | |
|---|---|---|
| 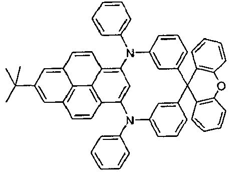 | 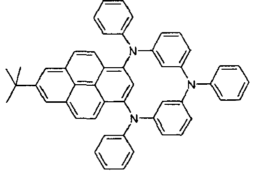 | 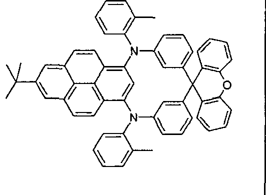 |
| (1) | (2) | (3) |
| 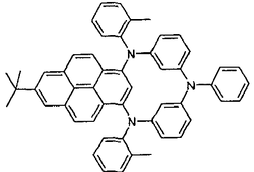 | 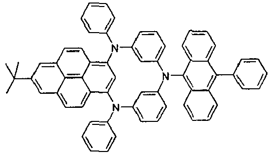 | 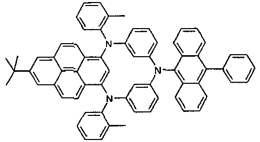 |
| (4) | (5) | (6) |
| 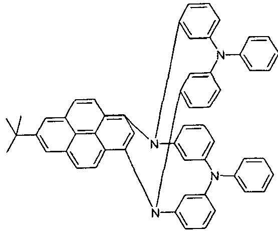 | 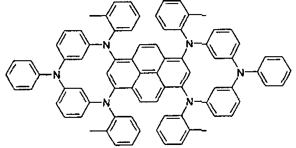 | 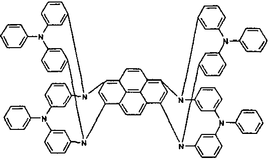 |
| (7) | (8) | (9) |
| 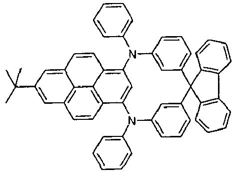 | 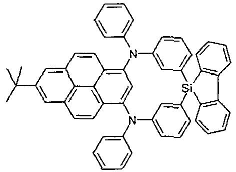 | 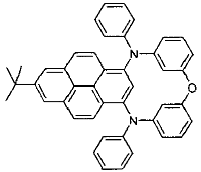 |
| (10) | (11) | (12) |
| 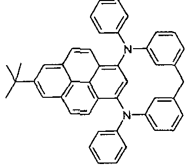 | 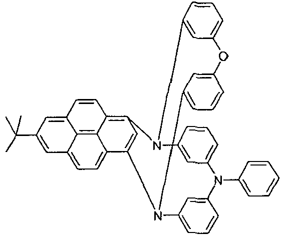 | 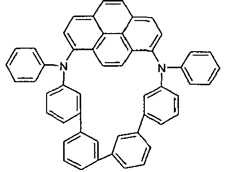 |
| (13) | (14) | (15) |
| 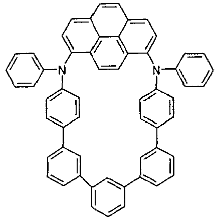 | 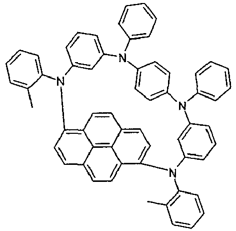 | 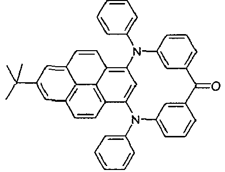 |
| (16) | (17) | (18) |
| 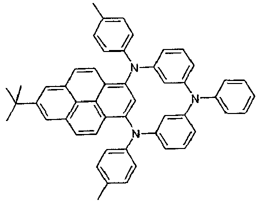 | 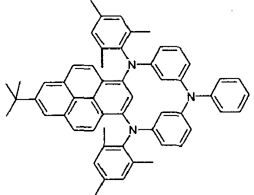 | 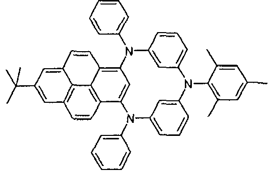 |
| (19) | (20) | (21 ) |
| 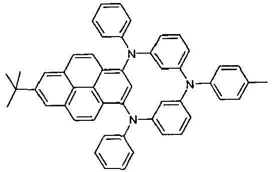 | 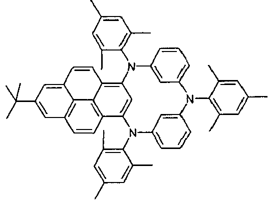 | 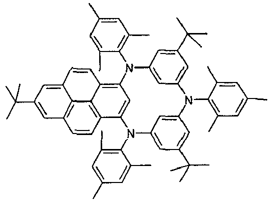 |
| (22) | (23) | (24) |
| 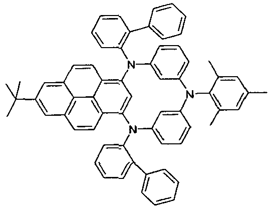 | 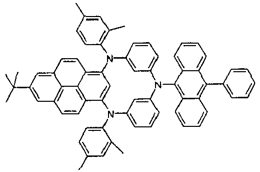 | 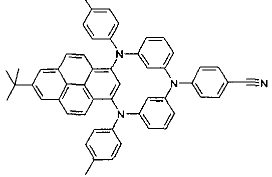 |
| (25) | (26) | (27) |
| 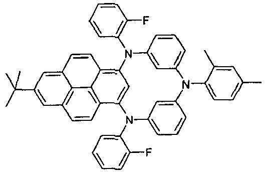 | 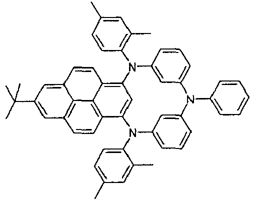 | 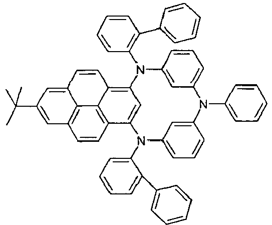 |
| (28) | (29) | (30) |
| 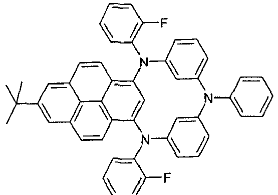 | 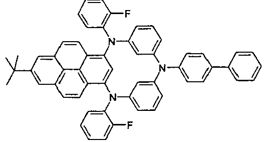 | 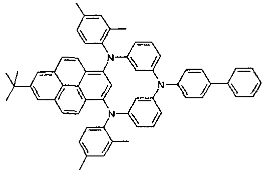 |
| (31) | (32) | (33) |
| 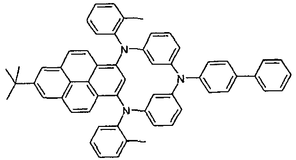 | 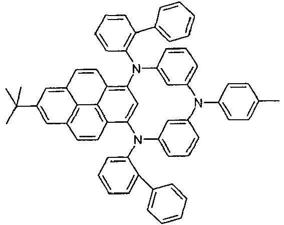 | 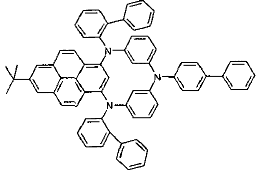 |
| (34) | (35) | (36) |
| 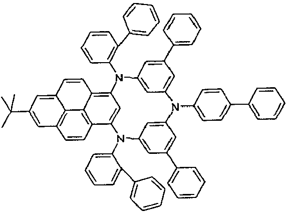 | 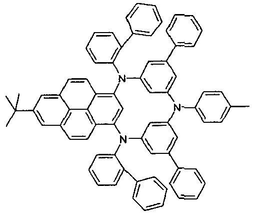 | 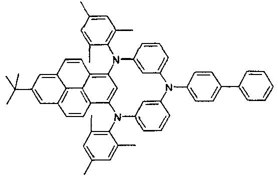 |
| (37) | (38) | (39) |
| 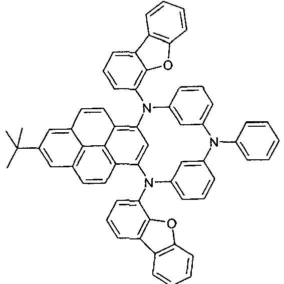 | 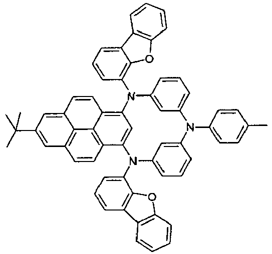 | 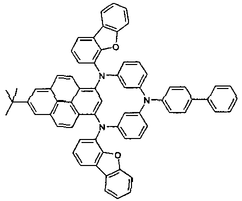 |
| (40) | (41) | (42) |
| 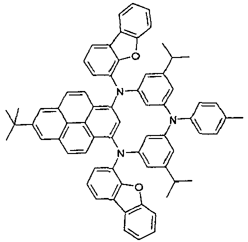 | 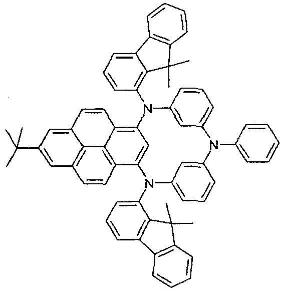 | 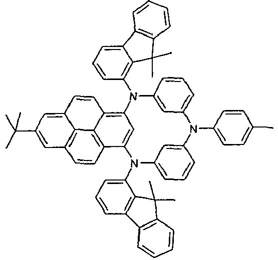 |
| (43) | (44) | (45) |
| 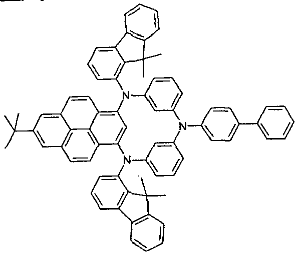 | 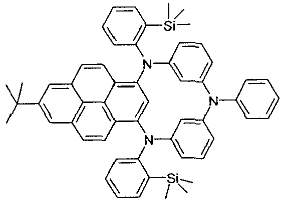 | 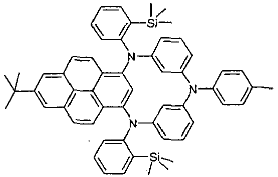 |
| (46) | (47) | (48) |
| 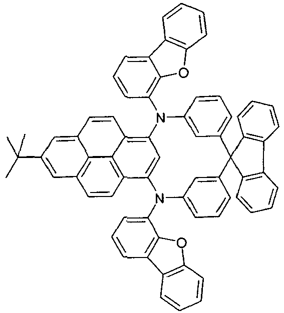 | 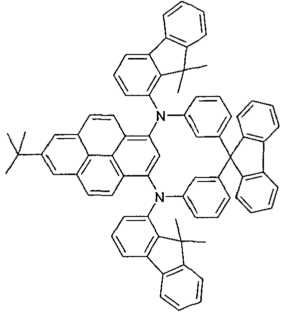 | 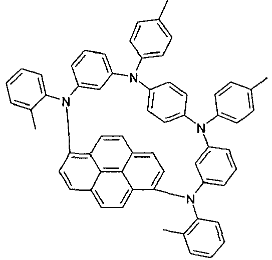 |
| (49) | (50) | (51) |
| 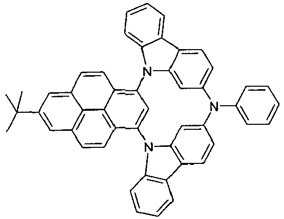 | 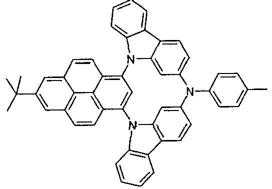 | 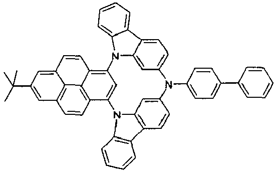 |
| (52) | (53) | (54) |
| 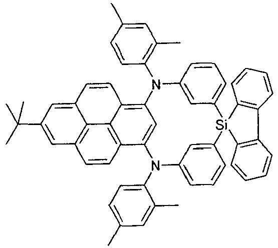 | 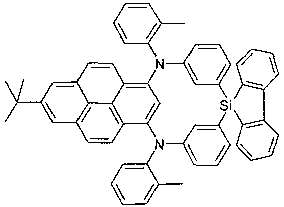 | 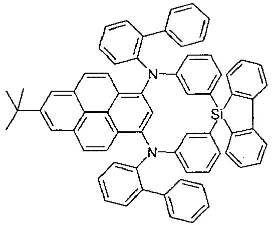 |
| (55) | (56) | (57) |
| 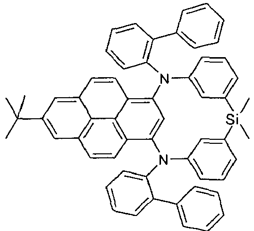 | 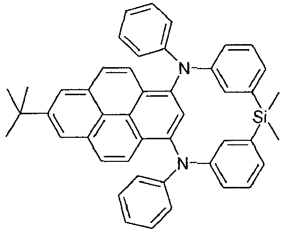 | 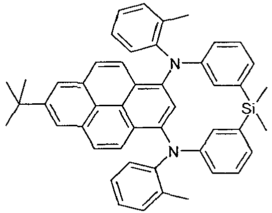 |
| (58) | (59) | (60) |
| 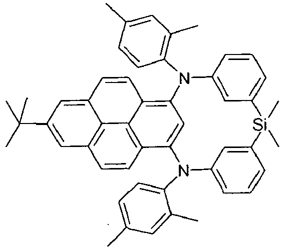 | 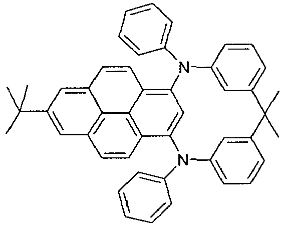 | 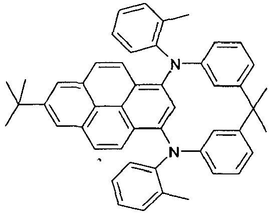 |
| (61) | (62) | (63) |
| 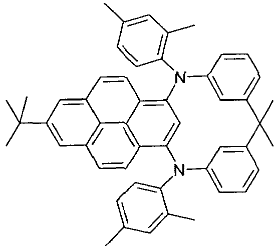 | 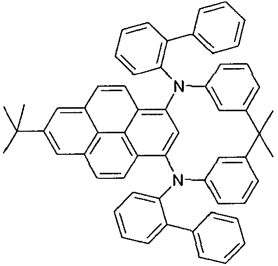 | 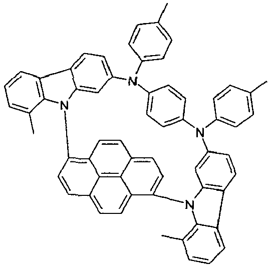 |
| (64) | (65) | (66) |
| 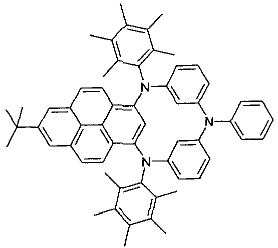 | 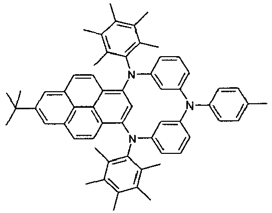 | 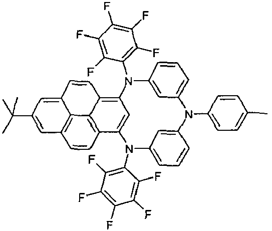 |
| (67) | (68) | (69) |
| 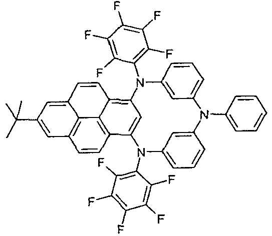 | 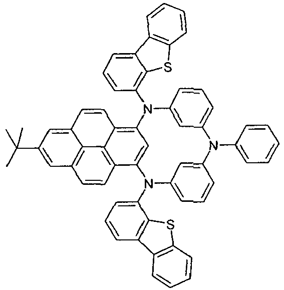 | 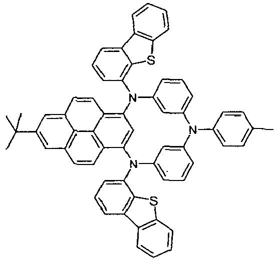 |
| (70) | (71) | (72) |
| 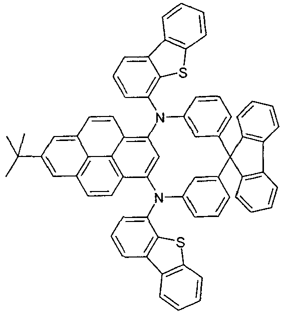 | 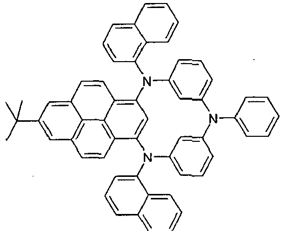 | 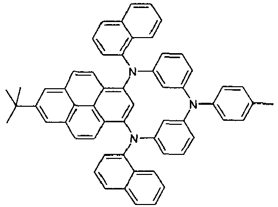 |
| (73) | (74) | (75) |
| 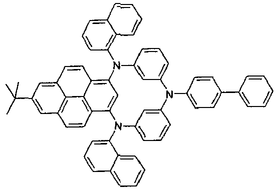 | 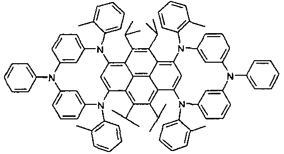 | 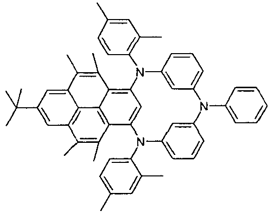 |
| (76) | (77) | (78) |
| 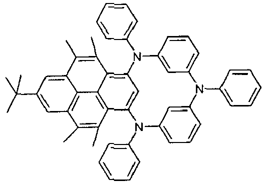 | 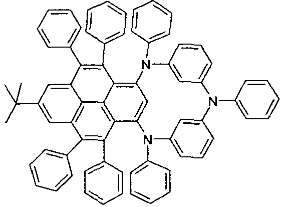 | 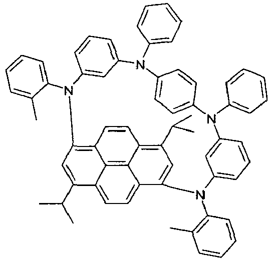 |
| (79) | (80) | (81) |
| 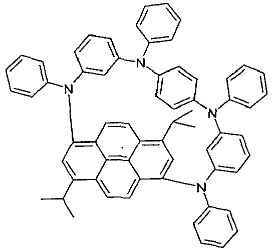 | 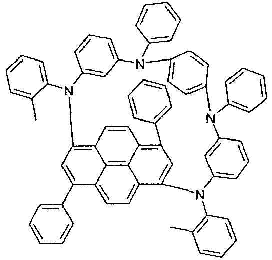 | 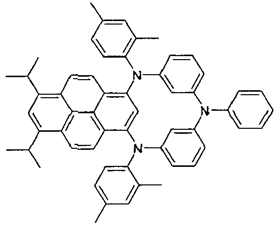 |
| (82) | (83) | (84) |
| 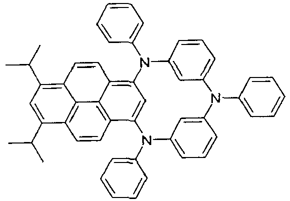 | 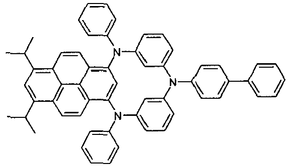 | 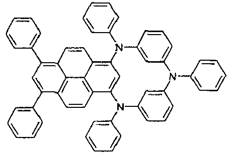 |
| (85) | (86) | (87) |
| 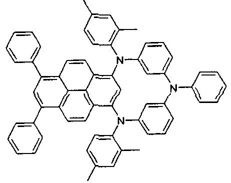 | 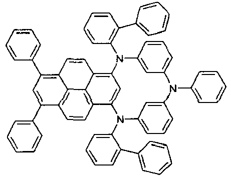 | 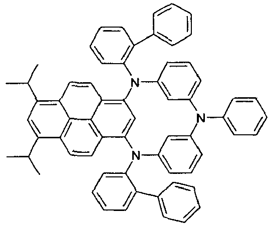 |
| (88) | (89) | (90) |
| 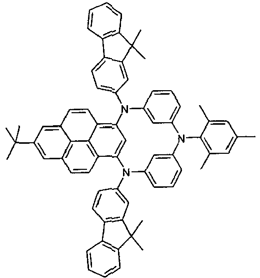 | 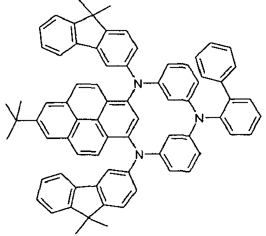 | 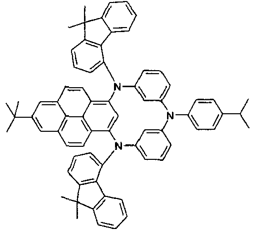 |
| (91) | (92) | (93) |

Gegenstand der vorliegenden Anmeldung ist weiterhin ein Verfahren zur Herstellung einer Verbindung gemäß Formel (I).

Bevorzugt ist ein Verfahren zur Herstellung einer Verbindung nach Formel (I), dadurch gekennzeichnet, dass es mindestens eine übergangsmetallkatalysierte Kupplungsreaktion umfasst. Bevorzugt ist die Kupplungsreaktion ausgewählt aus Buchwald-Kupplungsreaktionen. Bevorzugt ist einer der beiden Reaktionspartner der Buchwald-Kupplung ein Pyren-Derivat, das zwei oder mehr Aminogruppen trägt. Bevorzugt wird durch die Buchwald-Kupplung eine Verknüpfung zwischen zwei Aminogruppen, die an das Pyren-Derivat binden, eingeführt.

Bevorzugte Verfahren zur Herstellung der Verbindungen gemäß Formel (I) sind im Folgenden dargestellt. Dadurch werden dem Fachmann mögliche Syntheseverfahren vorgeschlagen, mit denen er die Verbindungen herstellen kann. Der Fachmann ist an die vorgestellten Verfahren jedoch nicht gebunden. Es kann sie nach Bedarf im Rahmen seines allgemeinen Fachwissens abwandeln, sofern dies erforderlich ist.

Nach einer Variante des Verfahrens werden zunächst zwei Monoarylaminogruppen an dem Pyrengrundkörper eingeführt, bevorzugt über eine Buchwald-Kupplung. Dabei entsteht ein Pyren-Derivat mit zwei sekundären Arylaminogruppen. In einem weiteren Schritt, bevorzugt einer Buchwald-Kupplung, wird dann eine Gruppe Ar¹-X-Ar¹ eingeführt, die die beiden Arylaminogruppen miteinander verbindet. Die Gruppe Ar¹-X-Ar¹ als reaktiver Baustein ist in wenigen Schritten herstellbar oder kommerziell erhältlich. Konkrete Beispiele für Synthesen dieser Gruppe sind in den Ausführungsbeispielen enthalten. Es können sich an den genannten Schritt optional weitere Funktionalisierungsschritte anschließen, in denen die Verbindung weiter modifiziert wird. Die Verbindung kann beliebig substituiert sein. 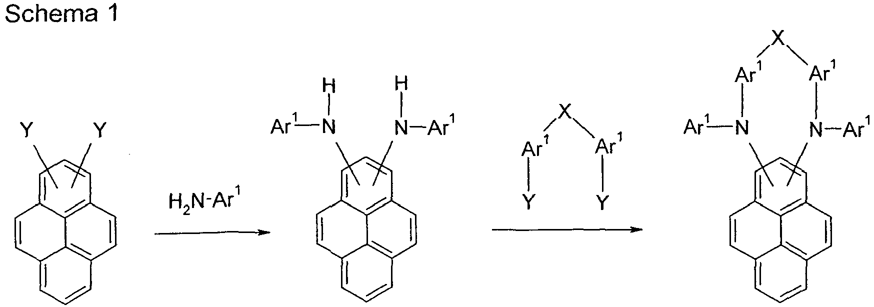
X = verbrückende Gruppe, bevorzugt CR₂, SiR₂, NR, O, S
Y = reaktive Gruppe, bevorzugt Cl, Br, I
Ar¹ = aromatisches oder heteroaromatisches Ringsystem
R = beliebiger organischer Rest

Nach einer anderen Variante werden zunächst zwei NH₂-Gruppen an dem Pyrengrundkörper eingeführt. Dabei entsteht ein Pyren-Derivat mit zwei primären Aminogruppen. In einem weiteren Schritt, bevorzugt einer Buchwald-Kupplung, wird dann eine Gruppe Ar¹-X-Ar¹, wie oben vorgestellt, eingeführt, die die beiden Arylaminogruppen miteinander verbindet. Es entsteht ein Pyren-Derivat mit zwei sekundären Arylaminogruppen, wobei die Arylgruppen miteinander verknüpft sind. In einem zweiten Schritt werden dann die sekundären Arylaminogruppen in tertiäre Arylaminogruppen überführt, bevorzugt durch eine zweite Buchwald-Kupplung mit einer weiteren Arylgruppe. Es können sich an den genannten Schritt optional weitere Funktionalisierungsschritte anschließen, in denen die Verbindung weiter modifiziert wird. Die Verbindung kann beliebig substituiert sein.

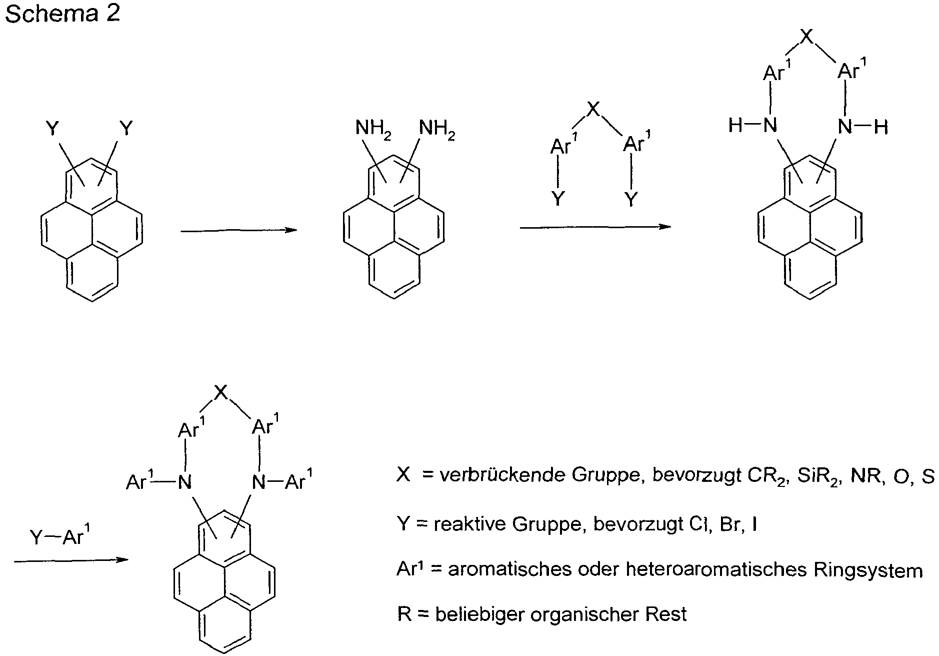

Die oben beschriebenen Verbindungen gemäß Formel (I), insbesondere Verbindungen, welche mit reaktiven Abgangsgruppen, wie Brom, lod, Chlor, Boronsäure oder Boronsäureester, substituiert sind, können als Monomere zur Erzeugung entsprechender Oligomere, Dendrimere oder Polymere Verwendung finden. Geeignete reaktive Abgangsgruppen sind beispielsweise Brom, lod, Chlor, Boronsäuren, Boronsäureester, Amine, Alkenyl- oder Alkinylgruppen mit endständiger C-C-Doppelbindung bzw. C-C-Dreifachbindung, Oxirane, Oxetane, Gruppen, die eine Cycloaddition, beispielsweise eine 1,3-dipolare Cycloaddition, eingehen, wie beispielsweise Diene oder Azide, Carbonsäurederivate, Alkohole und Silane.

Weiterer Gegenstand der Erfindung sind daher Oligomere, Polymere oder Dendrimere enthaltend eine oder mehrere Verbindungen gemäß Formel (I), wobei die Bindung(en) zum Polymer, Oligomer oder Dendrimer an beliebigen, in Formel (I) mit R¹, R² oder R³ substituierten Positionen lokalisiert sein können. Je nach Verknüpfung der Verbindung gemäß Formel (I) ist die Verbindung Bestandteil einer Seitenkette des Oligomers oder Polymers oder Bestandteil der Hauptkette. Unter einem Oligomer im Sinne dieser Erfindung wird eine Verbindung verstanden, welche aus mindestens drei Monomereinheiten aufgebaut ist. Unter einem Polymer im Sinne der Erfindung wird eine Verbindung verstanden, die aus mindestens zehn Monomereinheiten aufgebaut ist. Die erfindungsgemäßen Polymere, Oligomere oder Dendrimere können konjugiert, teilkonjugiert oder nichtkonjugiert sein. Die erfindungsgemäßen Oligomere oder Polymere können linear, verzweigt oder dendritisch sein. In den linear verknüpften Strukturen können die Einheiten gemäß Formel (I) direkt miteinander verknüpft sein oder sie können über eine bivalente Gruppe, beispielsweise über eine substituierte oder unsubstituierte Alkylengruppe, über ein Heteroatom oder über eine bivalente aromatische oder heteroaromatische Gruppe miteinander verknüpft sein. In verzweigten und dendritischen Strukturen können beispielsweise drei oder mehrere Einheiten gemäß Formel (I) über eine trivalente oder höhervalente Gruppe, beispielsweise über eine trivalente oder höhervalente aromatische oder heteroaromatische Gruppe, zu einem verzweigten bzw. dendritischen Oligomer oder Polymer verknüpft sein.

Für die Wiederholeinheiten gemäß Formel (I) in Oligomeren, Dendrimeren und Polymeren gelten dieselben Bevorzugungen wie oben für Verbindungen gemäß Formel (I) beschrieben.

Zur Herstellung der Oligomere oder Polymere werden die erfindungsgemäßen Monomere homopolymerisiert oder mit weiteren Monomeren copolymerisiert. Geeignete und bevorzugte Comonomere sind gewählt aus Fluorenen (z. B. gemäß EP 842208 oder WO 00/22026), Spirobifluorenen (z. B. gemäß EP 707020, EP 894107 oder WO 06/061181), Paraphenylenen (z. B. gemäß WO 1992/18552), Carbazolen (z. B. gemäß WO 04/070772 oder WO 2004/113468), Thiophenen (z. B. gemäß EP 1028136), Dihydrophenanthrenen (z. B. gemäß WO 2005/014689 oder WO 2007/006383), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere enthalten üblicherweise noch weitere Einheiten, beispielsweise emittierende (fluoreszierende oder phosphoreszierende) Einheiten, wie z. B. Vinyltriarylamine (z. B. gemäß WO 2007/068325) oder phosphoreszierende Metallkomplexe (z. B. gemäß WO 2006/003000), und/oder Ladungstransporteinheiten, insbesondere solche basierend auf Triarylaminen.

Die erfindungsgemäßen Polymere, Oligomere und Dendrimere weisen vorteilhafte Eigenschaften, insbesondere hohe Lebensdauern, hohe Effizienzen und gute Farbkoordinaten auf.

Die erfindungsgemäßen Polymere und Oligomere werden in der Regel durch Polymerisation von einer oder mehreren Monomersorten hergestellt, von denen mindestens ein Monomer im Polymer zu Wiederholungseinheiten der Formel (I) führt. Geeignete Polymerisationsreaktionen sind dem Fachmann bekannt und in der Literatur beschrieben. Besonders geeignete und bevorzugte Polymerisationsreaktionen, die zu C-C- bzw. C-N-Verknüpfungen führen, sind folgende:
(A) SUZUKI-Polymerisation;
(B) YAMAMOTO-Polymerisation;
(C) STILLE-Polymerisation; und
(D) HARTWIG-BUCHWALD-Polymerisation.

Wie die Polymerisation nach diesen Methoden durchgeführt werden kann und wie die Polymere dann vom Reaktionsmedium abgetrennt und aufgereinigt werden können, ist dem Fachmann bekannt und in der Literatur, beispielsweise in WO 2003/048225, WO 2004/037887 und WO 2004/037887, im Detail beschrieben.

Gegenstand der vorliegenden Erfindung ist somit auch ein Verfahren zur Herstellung der erfindungsgemäßen Polymere, Oligomere und Dendrimere, das dadurch gekennzeichnet ist, dass sie durch Polymerisation gemäß SUZUKI, Polymerisation gemäß YAMAMOTO, Polymerisation gemäß STILLE oder Polymerisation gemäß HARTWIG-BUCHWALD hergestellt werden. Die erfindungsgemäßen Dendrimere können gemäß dem Fachmann bekannten Verfahren oder in Analogie dazu hergestellt werden. Geeignete Verfahren sind in der Literatur beschrieben, wie z. B. in Frechet, Jean M. J.; Hawker, Craig J., "Hyperbranched polyphenylene and hyperbranched polyesters: new soluble, three-dimensional, reactive polymers", Reactive & Functional Polymers (1995), 26(1-3), 127-36; Janssen, H. M.; Meijer, E. W., "The synthesis and characterization of dendritic molecules", Materials Science and Technology (1999), 20 (Synthesis of Polymers), 403-458; Tomalia, Donald A., "Dendrimer molecules", Scientific American (1995), 272(5), 62-6; WO 2002/067343 A1 und WO 2005/026144 A1.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrof, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethyl-ether, Diethylenglycofdibutylether, Triethylenglycoldimethylether, Diethylenglycolmonobutylether, Tripropylenglycoldimethylether, Tetraethylenglycoldimethylether, 2-Isopropylnaphthalin, Pentylbenzol, Hexylbenzol, Heptylbenzol, Octylbenzol, 1,1-Bis(3,4-Dimethylphenyl)ethan oder Mischungen dieser Lösemittel.

Gegenstand der Erfindung ist daher weiterhin eine Formulierung, insbesondere eine Lösung, Dispersion oder Emulsion, enthaltend mindestens eine Verbindung gemäß Formel (I) oder mindestens ein Polymer, Oligomer oder Dendrimer enthaltend mindestens eine Einheit gemäß Formel (I) sowie mindestens ein Lösungsmittel, bevorzugt ein organisches Lösungsmittel. Wie solche Lösungen hergestellt werden können, ist dem Fachmann bekannt und beispielsweise in WO 2002/072714, WO 2003/019694 und der darin zitierten Literatur beschrieben.

Die erfindungsgemäßen Verbindungen gemäß Formel (I) eignen sich für den Einsatz in elektronischen Vorrichtungen, insbesondere in organischen Elektrolumineszenzvorrichtungen (OLEDs). Abhängig von der Substitution werden die Verbindungen in unterschiedlichen Funktionen und Schichten eingesetzt.

Weiterer Gegenstand der Erfindung ist daher die Verwendung einer Verbindung gemäß Formel (I) in einer elektronischen Vorrichtung. Dabei ist die elektronische Vorrichtung bevorzugt ausgewählt aus der Gruppe bestehend aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und besonders bevorzugt organischen Elektrolumineszenzvorrichtungen (OLEDs).

Weiterer Gegenstand der Erfindung ist eine elektronische Vorrichtung enthaltend mindestens eine Verbindung der Formel (I). Bevorzugt ist die elektronische Vorrichtung gewählt aus den oben angegebenen Vorrichtungen. Besonders bevorzugt ist eine organische Elektrolumineszenzvorrichtung, enthaltend Anode, Kathode und mindestens eine emittierende Schicht, dadurch gekennzeichnet, dass mindestens eine organische Schicht mindestens eine Verbindung gemäß Formel (I) enthält.

Außer Kathode, Anode und der emittierenden Schicht kann die organische Elektrolumineszenzvorrichtung noch weitere Schichten enthalten. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Excitonenblockierschichten, Zwischenschichten (Interlayers), Ladungserzeugungsschichten (Charge-Generation Layers) (IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer*)* und/oder organischen oder anorganischen p/n-Übergängen. Es sei aber darauf hingewiesen, dass nicht notwendigerweise jede dieser Schichten vorhanden sein muss und die Wahl der Schichten immer von den verwendeten Verbindungen abhängt und insbesondere auch von der Tatsache, ob es sich um eine fluoreszierende oder phosphoreszierende Elektrolumineszenzvorrichtung handelt.

Die Abfolge der Schichten der organischen Elektrolumineszenzvorrichtung ist bevorzugt die folgende: Anode-Lochinjektionsschicht-Lochtransportschicht-emittierende Schicht-Elektronentransportschicht-Elektroneninjektionsschicht-Kathode. Es müssen jedoch nicht alle der genannten Schichten vorhanden sein, und/oder es können zusätzliche Schichten vorhanden sein.

Die erfindungsgemäße organische Elektrolumineszenzvorrichtung kann mehrere emittierende Schichten enthalten. Besonders bevorzugt weisen diese Emissionsschichten in diesem Fall insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues oder gelbes oder orangefarbenes oder rotes Licht emittieren. Insbesondere bevorzugt sind Dreischichtsysteme, also Systeme mit drei emittierenden Schichten, wobei bevorzugt mindestens eine dieser Schichten mindestens eine Verbindung gemäß Formel (I) enthält und wobei die drei Schichten blaue, grüne und orange oder rote Emission zeigen (für den prinzipiellen Aufbau siehe z. B. WO 2005/011013). Es soll angemerkt werden, dass sich für die Erzeugung von weißem Licht anstelle mehrerer farbig emittierender Emitterverbindungen auch eine einzeln verwendete Emitterverbindung eignen kann, welche in einem breiten Wellenlängenbereich emittiert.

Es ist bevorzugt, wenn die Verbindung gemäß Formel (I) in einer emittierenden Schicht vorliegt. Insbesondere eignet sich die Verbindung gemäß Formel (I) zur Verwendung als emittierende Verbindung in einer emittierenden Schicht.

Die erfindungsgemäße Verbindung eignet sich besonders zur Verwendung als blau emittierende Verbindung. Dabei kann die betreffende elektronische Vorrichtung eine einzige emittierende Schicht enthaltend die erfindungsgemäße Verbindung enthalten, oder sie kann zwei oder mehr emittierende Schichten enthalten. Die weiteren emittierenden Schichten können dabei eine oder mehrere erfindungsgemäße Verbindungen oder alternativ andere Verbindungen enthalten.

Wenn die erfindungsgemäße Verbindung als emittierende Verbindung in einer emittierenden Schicht eingesetzt wird, wird sie bevorzugt in Kombination mit einem oder mehreren Matrixmaterialien eingesetzt. Bevorzugt liegt dabei das Matrixmaterial in höherem Anteil vor als die emittierende Verbindung. Unter Matrixmaterial der emittierenden Schicht wird dabei im Sinne der vorliegenden Anmeldung ein Material verstanden, das keine emittierende Funktion hat.

Es können auch mehrere emittierende Verbindungen gleichzeitig in der emittierenden Schicht vorhanden sein. Bevorzugt ist dies jedoch nicht der Fall.

Gemäß einer Ausführungsform der Erfindung liegen zwei oder mehr Matrixverbindungen in der emittierenden Schicht vor (Mixed-Matrix-System), bevorzugt genau zwei. Bevorzugt stellt dabei eines der beiden Matrixmaterialien ein Material mit lochtransportierenden Eigenschaften und das andere Matrixmaterial ein Material mit elektronentransportierenden Eigenschaften dar.

Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Mixed-Matrix-Systeme können in phosphoreszierenden emittierenden Schichten und in fluoreszierenden emittierenden Schichten eingesetzt werden. Genauere Angaben zu Mixed-Matrix-Systemen sind unter anderem in WO 2010/108579 und in den noch nicht offengelegten Anmeldungen EP 13000014.4 und EP 13000015.1 enthalten.

Der Anteil der erfindungsgemäßen Verbindung in der Mischung der emittierenden Schicht beträgt bevorzugt zwischen 0.1 und 50.0 Vol.-%, besonders bevorzugt zwischen 0.5 und 20.0 Vol.-%, und ganz besonders bevorzugt zwischen 1.0 und 10.0 Vol.-%. Entsprechend beträgt der Anteil des Matrixmaterials bzw. der Matrixmaterialien bevorzugt zwischen 50.0 und 99.9 Vol.-%, besonders bevorzugt zwischen 80.0 und 99.5 Vol.-%, und ganz besonders bevorzugt zwischen 90.0 und 99.0 Vol.-%.

Bevorzugte Matrixmaterialien zur Verwendung in Kombination mit den erfindungsgemäßen Materialien sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 2004/081017), der lochleitenden Verbindungen (z. B. gemäß WO 2004/058911), der elektronenleitenden Verbindungen, insbesondere Ketone, Phosphinoxide, Sulfoxide, etc. (z. B. gemäß WO 2005/084081 und WO 2005/084082), der Atropisomere (z. B. gemäß WO 2006/048268), der Boronsäurederivate (z. B. gemäß WO 2006/117052) oder der Benzanthracene (z. B. gemäß WO 2008/145239). Besonders bevorzugte Matrixmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrixmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind.

Bevorzugte Matrixmaterialien zur Verwendung in Kombination mit der Verbindung der Formel (I) in der emittierenden Schicht sind in der folgenden Tabelle abgebildet.

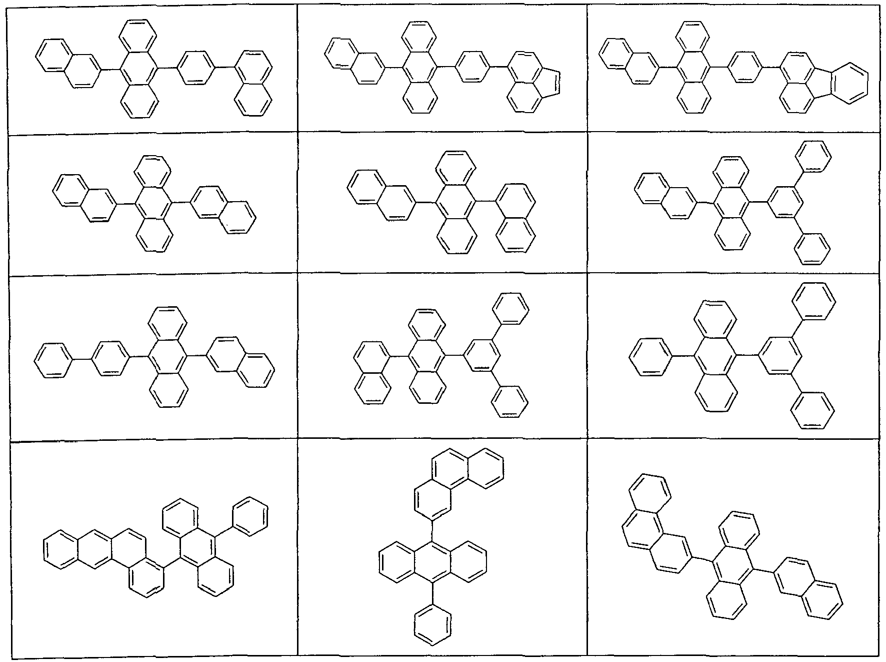

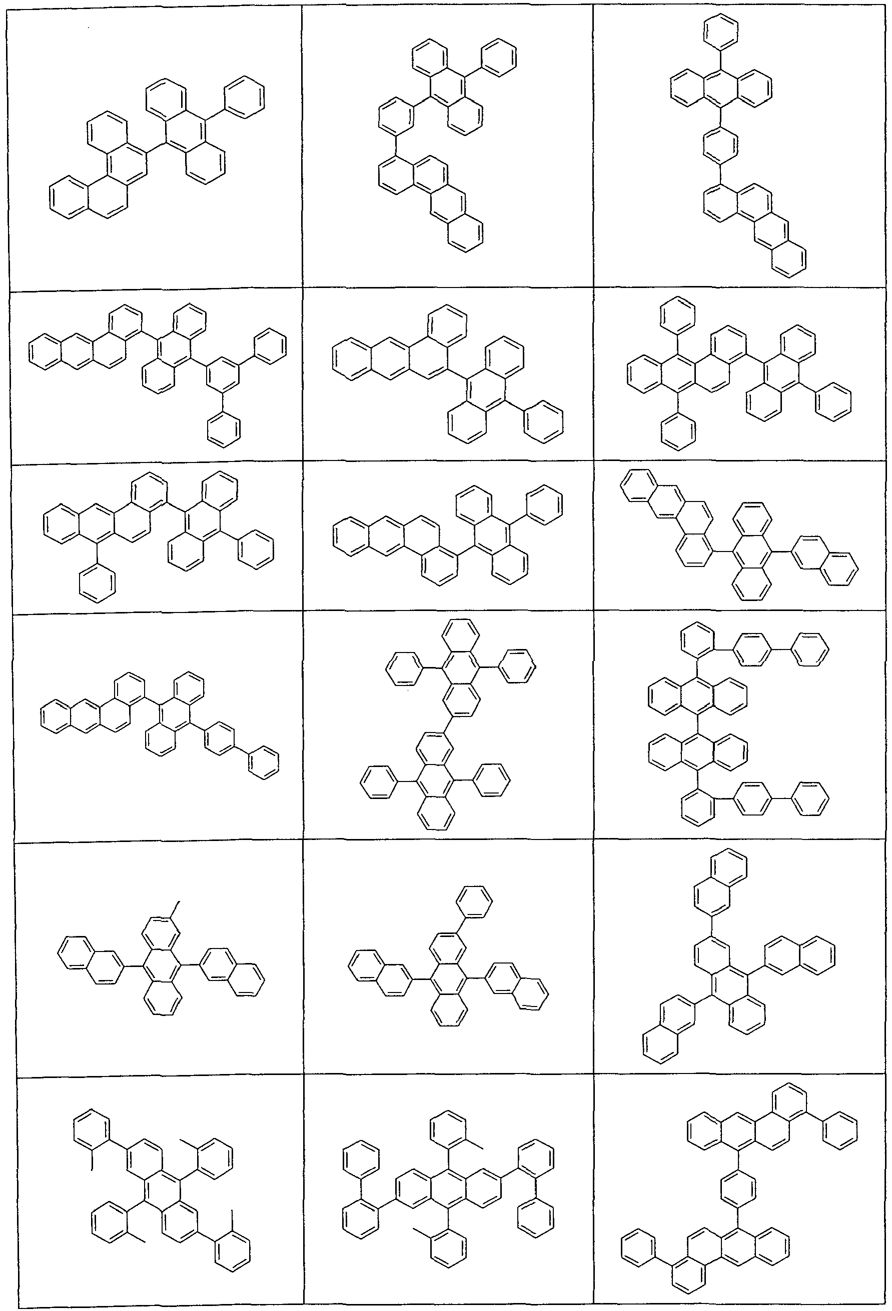

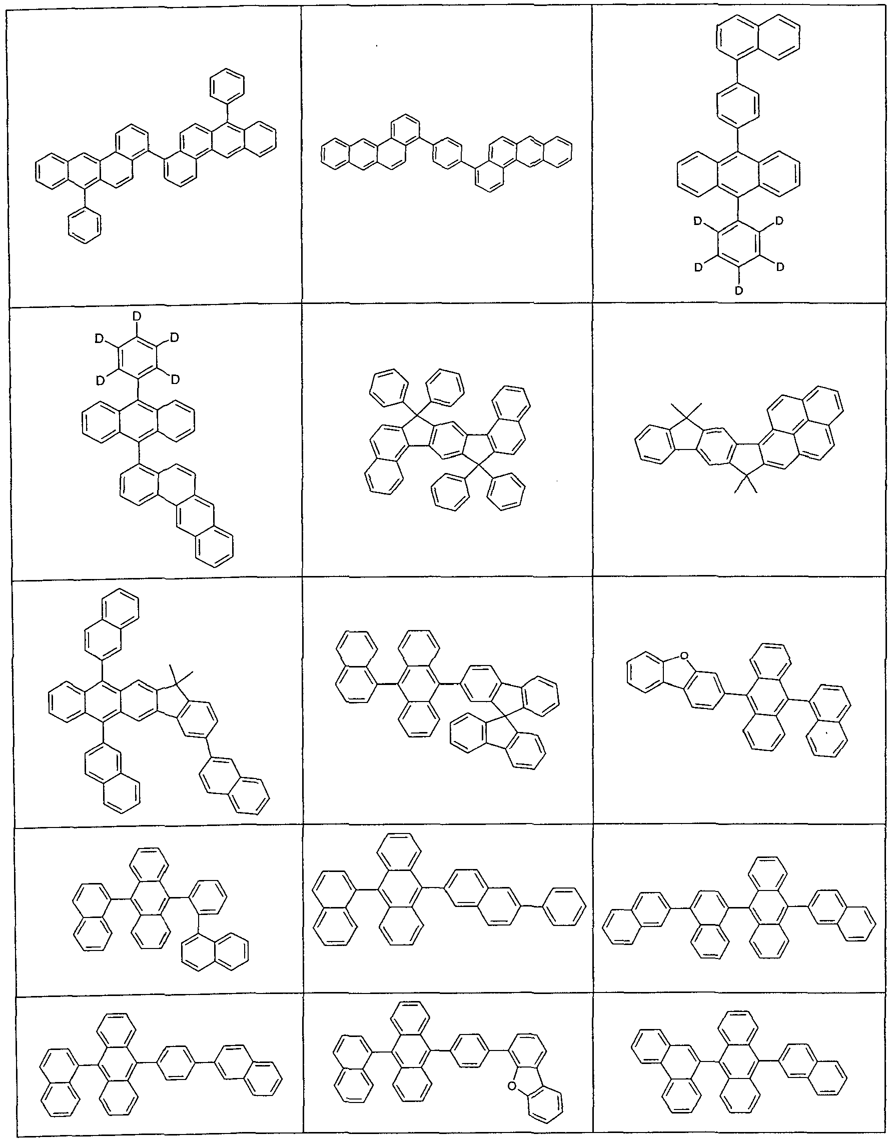

Die erfindungsgemäßen Verbindungen können auch in anderen Schichten eingesetzt werden, beispielsweise in einer Lochtransportschicht oder einer Lochinjektionsschicht.

Im Folgenden sind allgemein bevorzugte Materialklassen zur Verwendung als entsprechende Funktionsmaterialien in den erfindungsgemäßen organischen Elektrolumineszenzvorrichtungen aufgeführt.

Als phosphoreszierende Emitter eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende Emitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Dabei werden im Sinne der vorliegenden Erfindung alle lumineszierenden Iridium-, Platin- oder Kupferkomplexe als phosphoreszierende Verbindungen angesehen.

Beispiele der oben beschriebenen phosphoreszierenden Emitter können den Anmeldungen WO 2000/70655, WO 2001/41512, WO 2002/02714, WO 2002/15645, EP 1191613, EP 1191612, EP 1191614, WO 2005/033244, WO 2005/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind, zur Verwendung in den erfindungsgemäßen Vorrichtungen. Auch kann der Fachmann ohne erfinderisches Zutun weitere phosphoreszierende Komplexe in Kombination mit den erfindungsgemäßen Verbindungen in OLEDs einsetzen.

Bevorzugte fluoreszierende Emitter sind neben den erfindungsgemäßen Verbindungen ausgewählt aus der Klasse der Arylamine. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind.

Bevorzugte Matrixmaterialien zur Verwendung mit fluoreszierenden Emittern sind obenstehend aufgeführt.

Bevorzugte Matrixmaterialien für phosphoreszierende Emitter sind aromatische Amine, insbesondere Triarylamine, z. B. gemäß US 2005/0069729, Carbazolderivate (z. B. CBP, N,N-Biscarbazolylbiphenyl) oder Verbindungen gemäß WO 2005/039246, US 2005/0069729, JP 2004/288381, EP 1205527 oder WO 2008/086851, verbrückte Carbazolderivate, z. B. gemäß WO 2011/088877 und WO 2011/128017, Indenocarbazolderivate, z. B. gemäß WO 2010/136109 und WO 2011/000455, Azacarbazolderivate, z. B. gemäß EP 1617710, EP 1617711, EP 1731584, JP 2005/347160, Indolocarbazolderivate, z. B. gemäß WO 2007/063754 oder WO 2008/056746, Ketone, z. B. gemäß WO 2004/093207 oder WO 2010/006680, Phosphinoxide, Sulfoxide und Sulfone, z. B. gemäß WO 2005/003253, Oligophenylene, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Aluminiumkomplexe, z. B. BAIq, Diazasilol- und Tetraazasilol-Derivate, z. B. gemäß WO 2010/054729 und Diazaphosphol-Derivate, z. B. gemäß WO 2010/054730.

Geeignete Ladungstransportmaterialien, wie sie in der Lochinjektions- bzw. Lochtransportschicht bzw. Elektronenblockierschicht oder in der Elektronentransportschicht der erfindungsgemäßen organischen Elektrolumineszenzvorrichtung verwendet werden können, sind neben den erfindungsgemäßen Verbindungen beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden.

Beispiele für bevorzugte Lochtransportmaterialien, die in einer Lochtransport-, Lochinjektions- oder Elektronenblockierschicht in der erfindungsgemäßen Elektrolumineszenzvorrichtung verwendet werden können, sind Indenofluorenamine und Derivate (z. B. gemäß WO 06/122630 oder WO 06/100896), die in EP 1661888 offenbarten Aminderivate, Hexaazatriphenylenderivate (z. B. gemäß WO 01/049806), Aminderivate mit kondensierten Aromaten (z. B. gemäß US 5,061,569), die in WO 95/09147 offenbarten Aminderivate, Monobenzoindenofluorenamine (z. B. gemäß WO 08/006449) oder Dibenzoindenofluorenamine (z. B. gemäß WO 07/140847). Weiterhin geeignete Lochtransport- und Lochinjektionsmaterialien sind Derivate der oben abgebildeten Verbindungen, wie sie in JP 2001/226331, EP 676461, EP 650955, WO 01/049806, US 4780536, WO 98/30071, EP 891121, EP 1661888, JP 2006/253445, EP 650955, WO 06/073054 und US 5061569 offenbart werden.

Als Kathode der organischen Elektrolumineszenzvorrichtung sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-Zinn-Oxid (ITO) oder Indium-Zink Oxid (IZO). Bevorzugt sind weiterhin leitfähige, dotierte organische Materialien, insbesondere leitfähige dotierte Polymere.

Die Vorrichtung wird entsprechend (je nach Anwendung) strukturiert, kontaktiert und schließlich versiegelt, da sich die Lebensdauer der erfindungsgemäßen Vorrichtungen bei Anwesenheit von Wasser und/oder Luft verkürzt.

In einer bevorzugten Ausführungsform ist die erfindungsgemäße organische Elektrolumineszenzvorrichtung dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Dabei ist es jedoch auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al, Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (I) nötig. Hohe Löslichkeit lässt sich durch geeignete Substitution der Verbindungen erreichen.

Weiterhin bevorzugt ist es, dass zur Herstellung einer erfindungsgemäßen organischen Elektrolumineszenzvorrichtung eine oder mehrere Schichten aus Lösung und eine oder mehrere Schichten durch ein Sublimationsverfahren aufgetragen werden.

Erfindungsgemäß können die elektronischen Vorrichtungen enthaltend eine oder mehrere erfindungsgemäße Verbindungen in Displays, als Lichtquellen in Beleuchtungsanwendungen sowie als Lichtquellen in medizinischen und/oder kosmetischen Anwendungen (z.B. Lichttherapie) eingesetzt werden.

### Ausführungsbeispiele

### A) Synthese-Beispiele

Anilin, 1,3-Dibrombenzol, 1,2-Dibrombenzol, Phenol, N,N-Dimethylcarbamoylchlorid, 1-Brom-3-iod-benzol, 2-Brom-biphenyl, 1-Brombenzol, 2-Brom-1,3-dimethylbenzol, 1-Brom-2-fluorbenzol, o-Tolylamin, 4-Bromo-dibenzofuran, 2,4-Dimethylphenylamin und 2-Fluorphenylamin sind kommerziell erhältlich. Die Synthese von 1,3-Dibrom-7-tert-butyl-pyren ist in Adv. Mat, 2010, 22, 990-993 beschrieben.

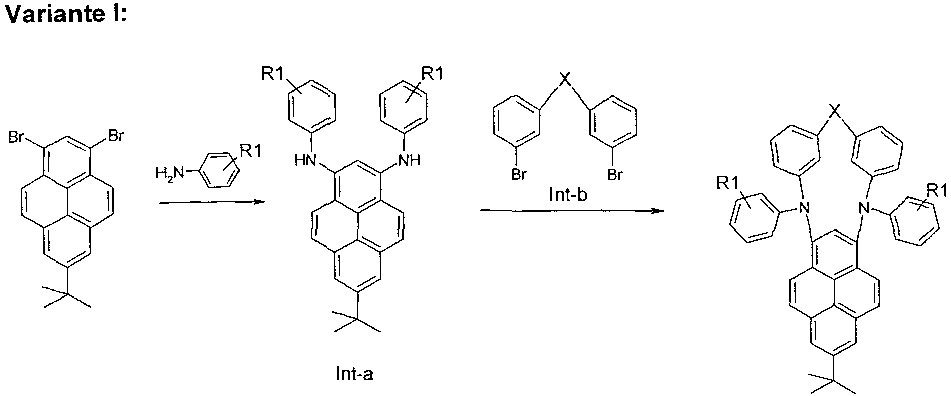

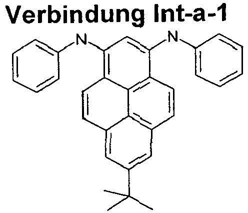

1,3-Dibrom-7-tert-butyl-pyren (20.0 g, 48.1 mmol) und Anilin (15.7 g, 168.2 mmol) werden in 300 mL Toluol gelöst. Danach wird Natrium-tert-butylat (13.9 g, 144 mmol) zugegeben. Die Lösung wird entgast und mit Argon gesättigt. Anschließend wird sie mit 1,1'-Bis(diphenylphosphino)-ferrocen (2.1 g, 3.85 mmol) und Palladium(II)-acetat (431 mg, 1.92 mmol) versetzt. Die Reaktionsmischung wird für 4h unter Schutzgasatmosphäre am Rückfluss erhitzt. Das Gemisch wird über Kieselgel und AlOx filtriert. Nach Filtration des Rohproduktes wird der verbleibende Rückstand aus einem Heptan/Toluol Gemisch umkristallisiert. Es werden 21.2 g (79 % d. Th.) des Produkts als gelber Feststoff erhalten.

Analog dazu werden folgende Verbindungen hergestellt:

| Verbindung | Pyren | Arylamin | Produkt |
|---|---|---|---|
| Int-a-2 | 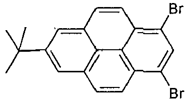 | 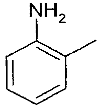 | 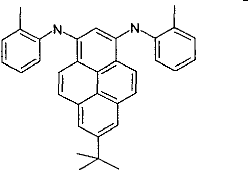 |
| Int-a-3 | 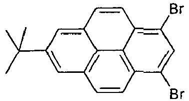 | 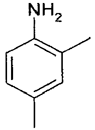 | 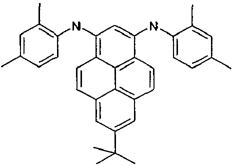 |
| Int-a-4 | 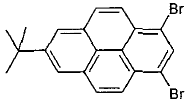 | 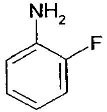 | 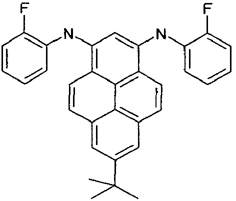 |

| Verbindung | Ausbeute |
|---|---|
| Int-a-2 | 91 % |
| Int-a-3 | 32 % |
| Int-a-4 | 41 % |

### Synthese der verbrückenden Gruppen Int-b-1 bis Int-b-3

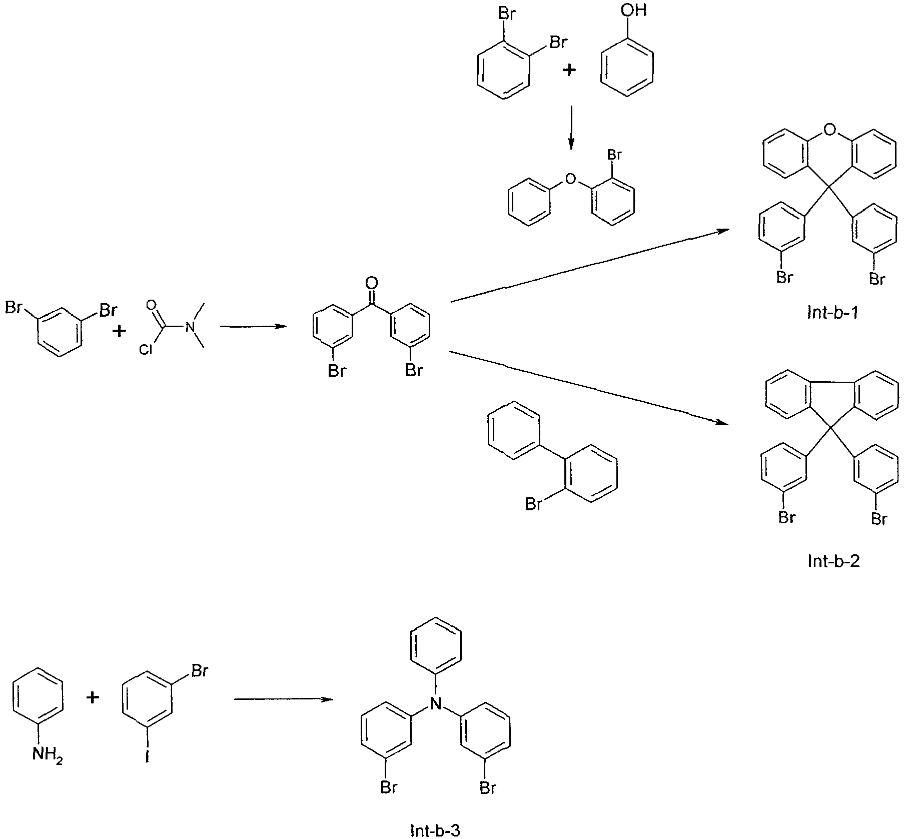

### Bis-(3-brom-phenyl)-methanon

1,3-Dibrombenzol (59.0 g, 250 mmol) wird in 1.1 L Diethylether gelöst und auf - 65°C abgekühlt. 100 mL n-BuLi Lösung (2.5 M in n-Hexan) wird langsam zugetropft und 1 h nachgerührt. N,N-Dimethylcarbamoylchlorid wird auf einmal unter starkem Rühren zugegeben. Das Gemisch wird 1 h bei - 65°C gerührt und dann langsam erwärmt. Anschliessend wird eine Mischung aus 250 mL Wasser und 25 mL Essigsäure langsam zugetropft. Das Produkt fällt als weißer Feststoff aus. Der Feststoff wird abgesaugt und mit Wasser und Ethanol nachgewaschen. Die Ausbeute beträgt 42.2 g (76 % d. Th) als weißer Feststoff.

### 1-Brom-2-phenoxy-benzol

1,2-Dibrom-benzol (451.1 g, 1.9 mol), Phenol (150.0 g, 1.50 mol), Kaliumcarbonat (132.2 g, 956 mmol) und Kupfer(I)oxid (273.7 g, 1.9 mol) werden gemischt und Glaskugeln werden zugegeben. Die Reaktionsmischung wird über Nacht bei 180°C Innentemperatur gerührt. Der Ansatz wird abgekühlt, und dann mit 300 mL DCM verdünnt. Die Ansatzlösung wird über einen Büchner-Trichter, und dann über Celite filtriert. Das Filtrat wird einrotiert. Das Öl wird unter Hochvakuum destilliert (1.5 mbar, Kopftemperatur 135°C). 168,1 g weißer Feststoff werden isoliert (45 % d. Th).

### 9,9-Bis-(3-bromo-phenyl)-9H-xanthen (Int-b-1)

Magnesium (3.17 g, 120.6 mmol) wird im Kolben mit einem Kristall Jod vorgelegt. 1-Bromo-2-phenoxy-benzol (23.2 g, 93.2 mmol), 190 mL wasserfreies THF, 26 mL 1,2-Dimethoxyethan und 0.78 mL 1,2-Dichloroethan werden in einem Tropftrichter vorgelegt. Das Eduktgemisch wird langsam zugetropft. Die Reaktion wird 3h bei 70°C nachgerührt und dann abgekühlt. Bis-(3-brom-phenyi)-methanon (32 g, 93.2 mmol) wird in 200 mL THF gelöst und langsam zugetropft. Die Reaktion wird 4h unter Rückfluss gerührt. Der Ansatz wird anschliessend filtriert und das THF wird abrotiert. 400 mL Essigsäure und 75 mL konzentrierte Salzsäure werden zugegeben und es wird 4h bei 75°C gerührt, bevor 200 mL Wasser zugegeben werden. Der helle Feststoff wird abgesaugt und mit Methanol gewaschen. Der Feststoff wird 1 h in siedendem Ethylacetat gerührt, filtriert und mit Ethanol gewaschen. Die Ausbeute beträgt 28.2 g (62 % d. Th) als weißer Feststoff.

### 9,9-Bis-(3-bromphenyl)-9H-fluoren (Int-b-2)

Magnesium (6.73 g, 255.8 mmol) wird im Kolben mit einem Kristall Jod vorgelegt. 2-Brombiphenyl (64.4 g, 276.3 mmol), 400 mL wasserfreies THF, 500 mL wasserfreies Toluol, 45 mL 1,2-Dimethoxyethan und 2.65 mL 1,2-Dichloroethan werden in einen Tropftrichter gegeben. Das Eduktgemisch wird langsam zugetropft. Die Reaktion wird 1h unter Rückfluss nachgerührt, und anschliessend abgekühlt. Bis-(3-brom-phenyl)-methanon (61.4 g, 180.6 mmol) wird in 600 mL THF gelöst und langsam zugetropft. Die Reaktion wird 5h unter Rückfluss gerührt. Der Ansatz wird filtriert und aus THF einrotiert. 500 mL Eisessig, 200 mL Essigsäure und 10 mL HBr werden zugegeben und es wird 64h unter Rückfluss gerührt. Der helle Feststoff wird abgesaugt und mit Ethylacetat und Ethanol gewaschen. Die Ausbeute beträgt 79.8 g (92 % d. Th) als weißer Feststoff.

### Bis-(3-brom-phenyl)-phenyl-amin (Int-b-3)

1-Brom-3-iodbenzol (200.5 g, 708.7 mmol), Anilin (30.0 g, 322 mmol), Kupfer-(1)-iodid (4.92 g, 25.8 mmol), Kaliumhydroxid (145.8 g, 2.58 mol) und 1,10-Phenanthrolin (4.67 g, 25.8 mmol) werden in 1L o-Xylol suspendiert. Die Lösung wird entgast und mit Argon gesättigt. Die Reaktionsmischung wird über Nacht unter Rückfluss gerührt. Das Gemisch wird über Kieselgel und AlOx mit Toluol filtriert und anschliessend einrotiert. Der hellbraune Feststoff wird abgesaugt. Der Feststoff wird dann säulenchromatographisch gereinigt (Heptan). Das Produkt wird in Heptan 3 h unter Rückfluss gerührt und dann filtriert. Die Ausbeute beträgt 58.6 g (45 % d. Th) als weißer Feststoff.

### 7-Tert-butyl-N,N'-diphenyt-pyren-1,3-diamin, verbrückt mit 9,9-Bis-(3-bromo-phenyl)-9H-xanthen (1)

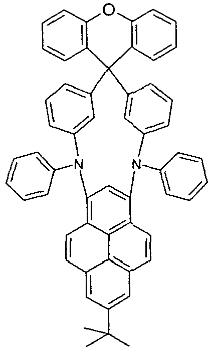

Natrium-tert-butylat (16.6 g, 173 mmol) wird in 500 mL Toluol suspendiert. Die Lösung wird entgast und mit Argon gesättigt. 7-tert-Butyl-N,N'-diphenyl-pyren-1,3-diamin (19.0 g, 43.1 mmol) und 9,9-Bis-(3-brom-phenyl)-9H-xanthen (21.2 g, 43.1 mmol) werden in 1.8 L THF gelöst, mit Argon gesättigt und in einen Tropftrichter gegeben. Danach werden Tri-tert-butylphosphin (1M-Lösung in Toluol,15.5 mL, 15.5 mmol) und Palladium(II)-acetat (2.32 g, 10.4 mmol) zur Base/Toluol-Lösung gegeben und die Mischung wird unter Rückfluss erhitzt. Anschliessend wird die Eduktmischung zugetropft. Die Reaktion wird 2h nachgerührt. Der Ansatz wird zweimal über Kieselgel und AlOx filtriert und einrotiert. Der Feststoff wird mit Toluol soxhletiert und in Toluol umkristallisiert. Die Ausbeute beträgt 1.9 g (6 % d. Th) als gelber Feststoff.

Analog dazu werden folgende Verbindungen hergestellt:

| Beispiel-Verbindung | Edukt | 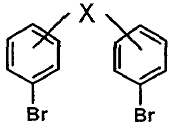 | Produkt | Ausbeute |
|---|---|---|---|---|
| 2 | Int-a-2 | 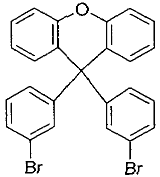 | 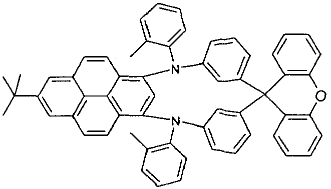 | 7% |
| 3 | Int-a-3 | 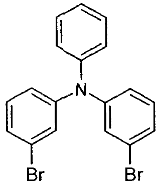 | 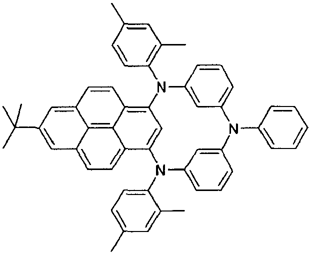 | 3% |
| 4 | Int-a-1 | 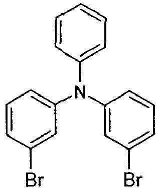 | 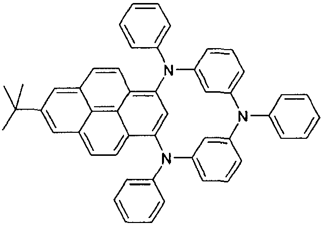 | 29% |
| 5 | Int-a-4 | 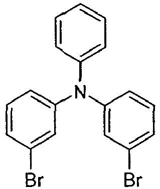 | 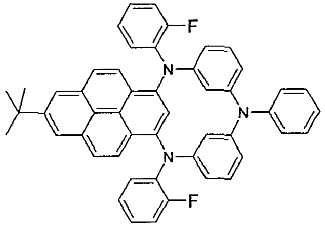 | 3% |
| 6 | Int-a-1 | 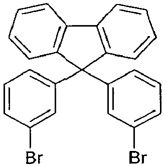 | 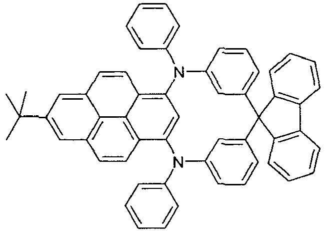 | 24% |

### Variante II:

### Synthese von N,N'-Bis-biphenyl-2-yl-7-tert-butyl-pyren-1,3-diamin verbrückt mit Triphenylamin (7)

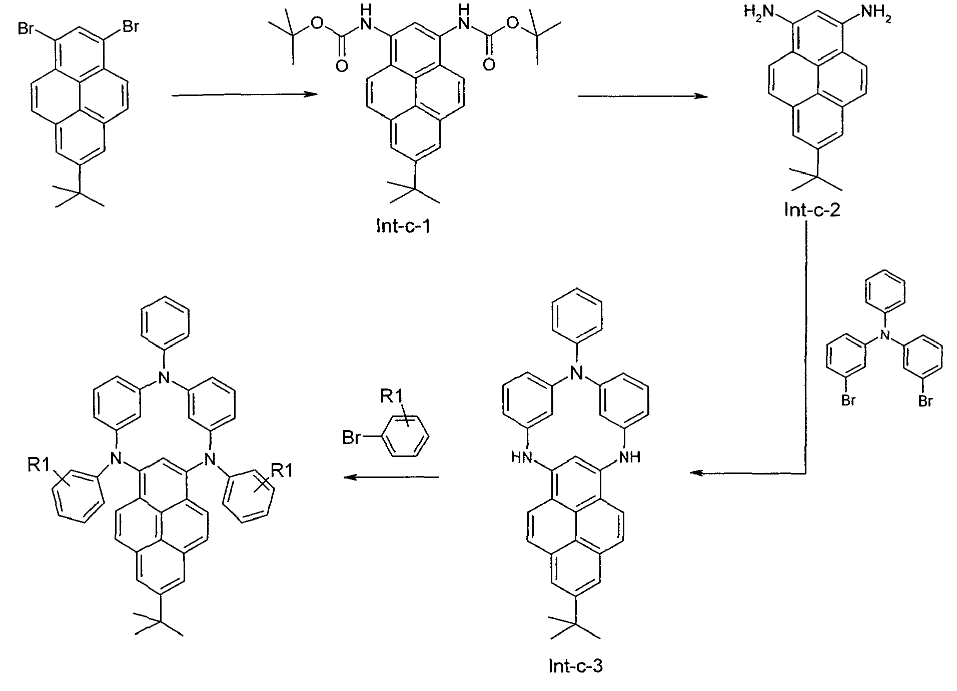

### (3-tert-Butoxycarbonylamino-7-tert-butylpyren-1-yl)carbamidsäure-tert-butylester (Int-c-1)

1,3-Dibrom-7-tert-butylpyren (5 g, 12 mmol), Carbamidsäure-tert-butylester (3.66 g, 31.2 mmol) und Cäsiumcarbonat (12.5 g, 38.5 mmol) werden in 100 mL wasserfreiem Dioxan suspendiert. Die Lösung wird entgast und mit Argon gesättigt. 9,9-Dimethyl-4,5-bis(diphenylphosphino)xanthen (556 mg, 0.96 mmol) und Palladium(II)acetat (162 mg, 0.72 mmol) werden zugegeben. Die Reaktion wird über Nacht unter Rückfluss gerührt. Die Reaktionsmischung wird filtriert und mit Heptan und dann Toluol soxhletiert. Das Produkt wird als brauner Feststoff erhalten: 2,6 g (46% d. Th.).

### 7-tert-Butyl-pyren-1,3-diamin (Int-c-2)

(3-tert-Butoxycarbonylamino-7-tert-butylpyren-1-yl)carbamidsäure-tert-butylester (5.9 g, 12 mmol) wird in 50 mL DCM gelöst. Trifluoressigsäure (11 g, 97 mmol) wird zugegeben. Der Ansatz wird 2h unter Rückfluss gerührt und anschließend eingeengt. Der Feststoff wird in Toluol gelöst und über AlOx filtriert. Das Produkt wird über eine Kieselgelsäule mit Heptan/Ethyl Acetat 1:1 eluiert. Die Ausbeute beträgt 615 mg (17.7% d. Th.) als schwarzer Feststoff.

### 7-tert-Butyl-pyren-1,3-diamin, verbrückt mit Triphenylamin (Int-c-3)

Natrium-*tert*.-butylat (615 mg, 6 mmol) wird in 60 mL Dioxan suspendiert. Die Lösung wird entgast und mit Argon gesättigt. 7-tert-Butyl-pyren-1,3-diamin (615 mg, 2.13 mmol) und Bis-(3-bromo-phenyl)-phenyl-amin (860 mg, 2.13 mmol) werden in 100 mL Dioxan gelöst, mit Argon gesättigt und im Tropftrichter vorgelegt. Danach werden S-Phos (52.6 mg, 0.128 mmol) und Palladium(II)-acetat (52.3 mg, 0.064 mmol) in der Base/Dioxan-Lösung zugegeben und die Reaktionsmischung bis zum Rückfluss erhitzt. Die Eduktmischung wird zugetropft. Die Reaktion wird 2 h nachgerührt. Der Ansatz wird zweimal über Kieselgel und AlOx filtriert und einrotiert. Die Ausbeute beträgt 883 mg (78.2 % d. Th) als schwarzes Öl.

### N,N'-Bis-biphenyl-2-yl-7-tert-butyl-pyren-1,3-diamin, verbrückt mit Triphenylamin (7)

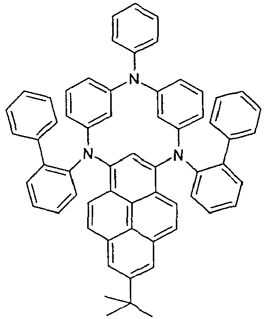

Das Edukt Int-c-3 (318 mg, 0.600 mmol), 2-Brom-biphenyl (490 mg, 2.1 mmol) und Natrium-tert.-butylat (173 mg, 1.8 mmol) werden in 30 mL Toluol suspendiert. Die Lösung wird entgast und mit Argon gesättigt. Danach werden Tri-tert-butylphosphin (1M-Lösung in Toluol,0.048 mL, 0.048 mmol) und Palladium(II)-acetat (11.6 mg, 0.024 mmol) zu der Reaktionsmischung gegeben und es wird unter Rückfluss erhitzt. Der Ansatz wird über Kieselgel und AlOx filtriert. Der Feststoff wird über eine Kieselgelsäule mit Heptan/ Ethyl Acetat 5:1 eluiert. Die Ausbeute beträgt 60 mg (12% d. Th) als gelber Feststoff.

Analog dazu werden folgende Verbindungen hergestellt:

| Beispiel-Verbindung | Edukt 1 | Edukt 2 | Produkt | Ausbeute |
|---|---|---|---|---|
| 8 | 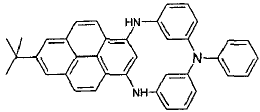 | 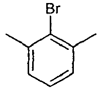 | 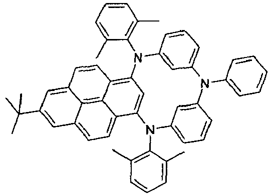 | 16% |
| 9 | 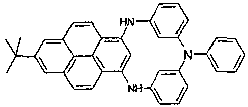 | 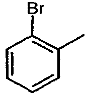 | 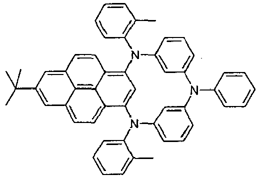 | 21% |
| 10 | 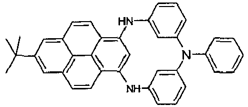 | 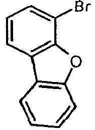 | 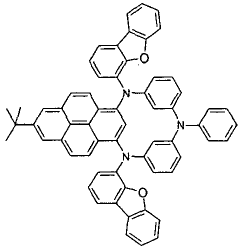 | 4% |

### B) Messung der Temperaturstabilität

Das Verhalten der erfindungsgemäßen Verbindung 4 bei hohen Temperaturen wird untersucht. Um festzustellen, wie hoch die Temperaturstabilität ist, werden TGA-Messungen unter Schutzgas von der Verbindung 4 und weiterhin zum Vergleich von der Verbindung aus dem Stand der Technik SEBV1 durchgeführt (für die Strukturen der Verbindungen vgl. Synthesebeispiele und Tabelle 1 unten).

Beide Proben werden mit einem Gerät TGA Q 5000 der Firma TA Instruments gemessen. Beide Proben werden in Stickstoffatmosphäre zwischen Raumtemperatur und 600°C mit einer Heizrate von 20°C pro Minute erhitzt.

Für die Verbindung 4 wird dabei als Temperatur Tₓ, bei der die Masse um 5% abgenommen hat, 451 °C gemessen.

Für die Vergleichsverbindung SEBV1 wird unter denselben Bedingungen eine Temperatur Tₓ von 401 °C gemessen.

Die Temperaturstabilität ist also für die erfindungsgemäße Verbindung 4 deutlich besser als für die Referenzverbindung SEBV1.

### C) Photolumineszenz-Messungen

Es werden Photolumineszenz-Spektren bei Raumtemperatur der erfindungsgemäßen Verbindung 3 und der im Stand der Technik bekannten Verbindung SEBV2 in Toluol aufgenommen (Konzentration 10⁻⁵g/mol). Für die Strukturen der Verbindungen vgl. Tabelle 1 unten.

Für die erfindungsgemäße Verbindung 3 wird eine CIE y-Koordinate von 0.077 gemessen, ein Maximum der Emission bei 447 nm, und eine Breite der Emissionsbande bei halber Intensität verglichen mit dem Maximumwert (FWHM) von 44 nm.

Für die Vergleichs-Verbindung SEBV2 wird eine CIE y-Koordinate von 0.090 gemessen, ein Maximum der Emission bei 450 nm, und eine Breite der Emissionsbande bei halber Intensität verglichen mit dem Maximumwert (FWHM) von 45 nm.

Es wird also für die erfindungsgemäße Verbindung 3 ein deutlich tiefblauerer Farbeindruck (CIE y) bei leicht kürzerwelliger Emission und leicht geringerer Breite der Emissionsbande gemessen, verglichen mit der Verbindung gemäß dem Stand der Technik SEBV2.

### D) Device-Beispiete

In den folgenden Beispielen werden die Daten verschiedener OLEDs vorgestellt. Die Herstellung von erfindungsgemäßen OLEDs sowie OLEDs nach dem Stand der Technik erfolgt nach einem allgemeinen Verfahren gemäß WO 04/058911, das auf die hier beschriebenen Gegebenheiten (Schichtdickenvariation, Materialien) angepasst wird.

Die OLEDs haben den folgenden Aufbau:
Glasplättchen, die mit strukturiertem ITO (Indium Zinn Oxid) der Dicke 50 nm beschichtet sind, werden zur verbesserten Prozessierung mit 20 nm PEDOT beschichtet (Poly(3,4-ethylendioxy-2,5-thiophen), aus Wasser aufgeschleudert; bezogen von H. C. Starck, Goslar, Deutschland). Diese beschichteten Glasplättchen bilden die Substrate, auf welche die OLEDs aufgebracht werden. Der allgemeine Schichtaufbau ist folgender: Substrat / Optionale Lochinjektionsschicht (HIL) / Lochtransportschicht (HTL) / Optionale Zwischenschicht (IL) / Elektronenblockierschicht (EBL) / Emissionsschicht (EML) / Optionale Lochblockierschicht (HBL) / Elektronentransportschicht (ETL) / Optionale Elektroneninjektionsschicht (EIL) und abschließend eine Kathode. Die Kathode wird durch eine 100 nm dicke Aluminiumschicht gebildet.

Der genaue Aufbau der OLEDs ist den Beispielen zu entnehmen. Die zur Herstellung der OLEDs benötigten Materialien sind in Tabelle 1 gezeigt.

Alle Materialien werden in einer Vakuumkammer thermisch aufgedampft. Dabei besteht die Emissionsschicht immer aus mindestens einem Matrixmaterial und einer emittierenden Verbindung, der dem Matrixmaterial bzw. den Matrixmaterialien durch Coverdampfung in einem bestimmten Volumenanteil beigemischt wird. Eine Angabe wie H1 (95%):SEBV1 (5%) bedeutet hierbei, dass das Material SEBV1 in einem Volumenanteil von 5% und H1 in einem Anteil von 95% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in Im/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektrum werden bei einer Leuchtdichte von 1000 cd/m² bestimmt und daraus die CIE 1931 x und y Farbkoordinaten berechnet. Als Lebensdauer LD80 @ 60 mA wird die Zeit definiert, nach der die Leuchtdichte bei einem Betrieb mit konstantem Strom von 60 mA/cm² auf 80% abgesunken ist. Die Werte für die Lebensdauer können mit Hilfe dem Fachmann bekannten Umrechnungsformeln auf eine Angabe für andere Startleuchtdichten umgerechnet werden.

Im folgenden werden die Beispiele näher erläutert, um die Eigenschaften und Vorteile der erfindungsgemäßen OLEDs zu verdeutlichen. Bereits die Verbesserung eines einzelnen Parameters kann dabei einen signifikanten Fortschritt darstellen, da unterschiedliche Anwendungen unterschiedliche Anforderungen bezüglich einzelner Parameter stellen.

Der erfindungsgemäßen Materialien 1 und 4 werden als Emitter in blau emittierenden OLEDs getestet. Weiterhin wird eine OLED enthaltend den im Stand der Technik bekannten Emitter SEBV1 hergestellt.

Als Testaufbau wird der folgende Aufbau verwendet: ITO/PEDOT (20 nm)/HTM1(140 nm)/HIL1(5 nm)/ HTM2(20 nm)/H1:x% Emitter (20 nm)/ETM1:50 % LiQ(30 nm)/Al.

Die erfindungsgemäße Verbindung 1 zeigt in obigen Testbauteil bei 3%(5%)-iger Dotierung bei 1000 cd/m² eine Farbkoordinate CIE x/y von 0,137/0,100, (0,135/0,105), eine EQE von 5,8% (6,4%) und eine Lebensdauer LD80 von 25 h (20 h).

Die erfindungsgemäße Verbindung 4 weist unter denselben Bedingungen bei 3%(5%)-iger Dotierung eine Farbkoordinate CIE x/y von 0,133/0,118, (0,134/0,117), eine EQE von 6,6% (6,8%) und eine Lebensdauer LD80 von 47 h (31 h) auf.

Der Referenzemitter SEBV1 zeigt bei 3%(5%)-iger Dotierung bei 1000cd/m² eine Farbkoordinate CIE x/y von 0,137/0,102 (0,136/0,109), eine EQE von 5,7% (5,8%) und eine Lebensdauer LD80 von 27h (31 h).

Die erfindungsgemäßen Materialien ergeben bei Einsatz als Emitter in OLEDs somit Verbesserungen gegenüber dem Stand der Technik in allen Parametern, vor allem bezüglich Lebensdauer und Effizienz.

Tabelle 1: Strukturformeln der verwendeten Materialien für die OLEDs

| | |
|---|---|
| 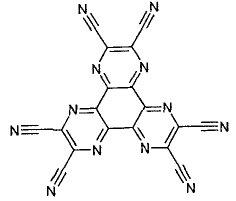 | 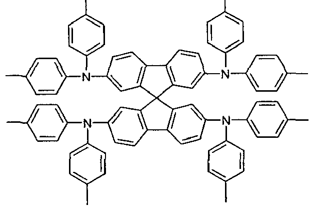 |
| HIL1 | HTM1 |
| 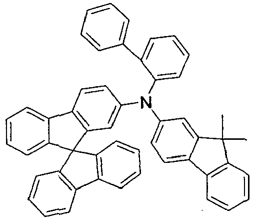 | 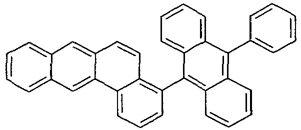 |
| HTM2 | H1 |
| 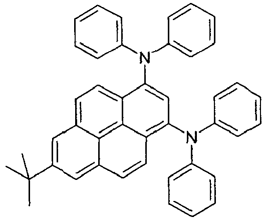 | 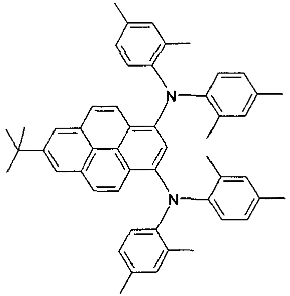 |
| SEBV1 | SEBV2 |
| 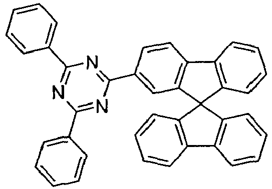 | 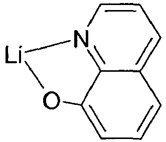 |
| ETM1 | LiQ |

## Patentansprüche

1. Verbindung einer Formel (I) 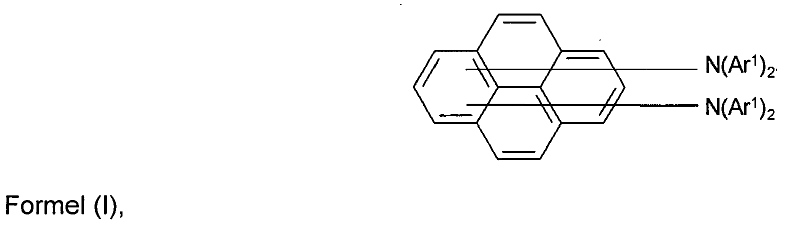 wobei die Pyrengruppe an jeder freien Position mit einem Rest R¹ substituiert sein kann, und wobei gilt:
in der Formel (I) ist mindestens eine Gruppe Ar¹, die Bestandteil einer Gruppe N(Ar¹)₂ ist, über eine Gruppe X mit einer anderen Gruppe Ar¹, die Bestandteil einer anderen Gruppe N(Ar¹)₂ ist, verknüpft;
Ar¹ ist bei jedem Auftreten gleich oder verschieden ein aromatisches oder heteroaromatisches Ringsystem mit 6 bis 30 aromatischen Ringatomen, das mit einem oder mehreren Resten R³ substituiert sein kann, wobei Gruppen Ar¹ dabei über Gruppen X verknüpft sein können;
X ist bei jedem Auftreten gleich oder verschieden eine Einfachbindung, oder eine divalente Gruppe gewählt aus einer Aryl- oder Heteroarylgruppe mit 6 bis 20 aromatischen Ringatomen, die mit einem oder mehreren Resten R² substituiert sein kann, BR², C(R²)₂, -R²C=CR²-, C(=O), Si(R²)₂, NR², PR², P(=O)R² O, S, S(=O), S(=O)₂, oder eine Kombination aus 2, 3, 4 oder 5 gleichen oder verschiedenen der genannten divalenten Gruppen;
R¹ ist bei jedem Auftreten gleich oder verschieden H, D, F, C(=O)R⁴, CN, Si(R⁴)₃, N(Ar¹)2, N(R⁴)₂, P(=O)(R⁴)₂, OAr¹, S(=O)R⁴, S(=O)₂R⁴, eine geradkettige Alkyl- oder Alkoxygruppe mit 1 bis 20 C-Atomen oder eine verzweigte oder cyclische Alkyl- oder Alkoxygruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁴ substituiert sein können und wobei eine oder mehrere CH₂-Gruppen in den oben genannten Gruppen durch -R⁴C=CR⁴-, -C≡C-, Si(R⁴)₂, C=O, C=NR⁴, -C(=O)O-, -C(=O)NR⁴-, NR⁴, P(=O)(R⁴), -O-, -S-, SO oder SO₂ ersetzt sein können, oder ein aromatisches oder heteroaromatisches Ringsystem mit 5 bis 30 aromatischen Ringatomen, das jeweils durch einen oder mehrere Reste R⁴ substituiert sein kann, wobei zwei oder mehr Reste R¹ miteinander verknüpft sein können und einen Ring bilden können;
R² ist bei jedem Auftreten gleich oder verschieden H, D, F, C(=O)R⁴, CN, Si(R⁴)₃, N(R⁴)₂, P(=O)(R⁴)₂, S(=O)R⁴, S(=O)₂R⁴, eine geradkettige Alkyl- oder Alkoxygruppe mit 1 bis 20 C-Atomen oder eine verzweigte oder cyclische Alkyl- oder Alkoxygruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁴ substituiert sein können und wobei eine oder mehrere CH₂-Gruppen in den oben genannten Gruppen durch - R⁴C=CR⁴-, -C≡C-, Si(R⁴)₂, C=O, C=NR⁴, -C(=O)O-, -C(=O)NR⁴-, NR⁴, P(=O)(R⁴), -O-, -S-, SO oder SO₂ ersetzt sein können, oder ein aromatisches oder heteroaromatisches Ringsystem mit 5 bis 30 aromatischen Ringatomen, das jeweils durch einen oder mehrere Reste R⁴ substituiert sein kann, wobei zwei oder mehr Reste R² miteinander verknüpft sein können und einen Ring bilden können;
R³ ist bei jedem Auftreten gleich oder verschieden H, D, F, C(=O)R⁴, CN, Si(R⁴)₃, N(R⁴)₂, P(=O)(R⁴)₂, S(=O)R⁴, S(=O)₂R⁴, eine geradkettige Alkyl- oder Alkoxygruppe mit 1 bis 20 C-Atomen oder eine verzweigte oder cyclische Alkyl- oder Alkoxygruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁴ substituiert sein können und wobei eine oder mehrere CH₂-Gruppen in den oben genannten Gruppen durch - R⁴C=CR⁴-, -C≡C-, Si(R⁴)₂, C=O, C=NR⁴, -C(=O)O-, -C(=O)NR⁴-, NR⁴, P(=O)(R⁴), -O-, -S-, SO oder SO₂ ersetzt sein können, oder ein aromatisches oder heteroaromatisches Ringsystem mit 5 bis 30 aromatischen Ringatomen, das jeweils durch einen oder mehrere Reste R⁴ substituiert sein kann, wobei zwei oder mehr Reste R³ miteinander verknüpft sein können und einen Ring bilden können;
R⁴ ist bei jedem Auftreten gleich oder verschieden H, D, F oder ein aliphatischer, aromatischer oder heteroaromatischer organischer Rest mit 1 bis 20 C-Atomen, in dem auch ein oder mehrere H-Atome durch D oder F ersetzt sein können; dabei können zwei oder mehr Substituenten R⁴ miteinander verknüpft sein und einen Ring bilden.

2. Verbindung nach Anspruch 1, **dadurch gekennzeichnet, dass** die Gruppe X bei jedem Auftreten gleich oder verschieden eine divalente Gruppe gewählt aus C(R²)₂, NR², O und S ist, nochmals stärker bevorzugt eine divalente Gruppe gewählt aus C(R²)₂ und NR² ist.

3. Verbindung nach Anspruch 1 oder 2, **dadurch gekennzeichnet, dass** in der Verbindung der Formel (I) genau eine oder genau 2 Gruppen X vorliegen, so dass genau 1 Paar von Gruppen Ar¹ oder genau 2 Paare von Gruppen Ar¹ miteinander über jeweils eine Gruppe X verknüpft sind.

4. Verbindung nach einem oder mehreren der Ansprüche 1 bis 3,
**dadurch gekennzeichnet, dass** Ar¹ bei jedem Auftreten gleich oder verschieden ein aromatisches Ringsystem mit 6 bis 12 aromatischen Ringatomen ist, das mit einem oder mehreren Resten R³ substituiert sein kann, und bevorzugt Phenyl ist, das mit einem oder mehreren Resten R³ substituiert sein kann.

5. Verbindung nach Anspruch 4, **dadurch gekennzeichnet, dass** Ar¹ eine Phenylgruppe ist, die mit einem oder mehreren Resten R³ substituiert sein kann, und dass die Bindung an die Gruppe X und an das Stickstoffatom an der Phenylgruppe in meta-Position zueinander stehen.

6. Verbindung nach einem oder mehreren der Ansprüche 1 bis 5,
**dadurch gekennzeichnet, dass** die Pyrengruppe mit mindestens einem Rest R¹ substituiert ist, der nicht gleich H ist.

7. Verbindung nach einem oder mehreren der Ansprüche 1 bis 6,
**dadurch gekennzeichnet, dass** die Pyrengruppe mit mindestens einem Rest R¹ substituiert ist, der gewählt ist aus einer geradkettigen Alkylgruppe mit 1 bis 8 C-Atomen oder einer verzweigten oder cyclischen Alkylgruppe mit 3 bis 8 C-Atomen, wobei die Alkylgruppen mit einem oder mehreren Resten R⁴ substituiert sein können, oder aus einer Aryl- oder Heteroarylgruppe mit 6 bis 14 aromatischen Ringatomen, die mit einem oder mehreren Resten R⁴ substituiert sein kann.

8. Verbindung nach einem oder mehreren der Ansprüche 1 bis 7,
**dadurch gekennzeichnet, dass** Reste R² an einer Gruppe X, die C(R²)₂ oder Si(R²)₂ darstellt, gewählt sind aus einer geradkettigen Alkylgruppe mit 1 bis 8 C-Atomen oder einer verzweigten oder cyclischen Alkylgruppe mit 3 bis 8 C-Atomen, wobei die Alkylgruppen mit einem oder mehreren Resten R⁴ substituiert sein können, oder aus einer Aryl- oder Heteroarylgruppe mit 6 bis 14 aromatischen Ringatomen, die mit einem oder mehreren Resten R⁴ substituiert sein kann.

9. Verbindung nach einem oder mehreren der Ansprüche 1 bis 8, **dadurch gekennzeichnet, dass** die Gruppen N(Ar¹)₂ in Formel (I) an Positionen gewählt aus den Positionen 1, 3, 6 und 8 des Pyrens gebunden sind.

10. Verbindung nach einem oder mehreren der Ansprüche 1 bis 9, **dadurch gekennzeichnet, dass** miteinander über Gruppen X verbundene Gruppen N(Ar¹)₂ an Positionen 1 und 3 des Pyrens gebunden sind.

11. Verbindung nach einem oder mehreren der Ansprüche 1 bis 10, **dadurch gekennzeichnet, dass** sie einer der Formeln (I-1) bis (I-5) entspricht: 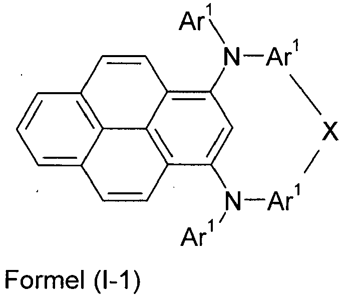 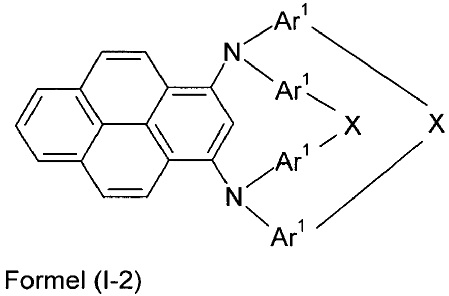 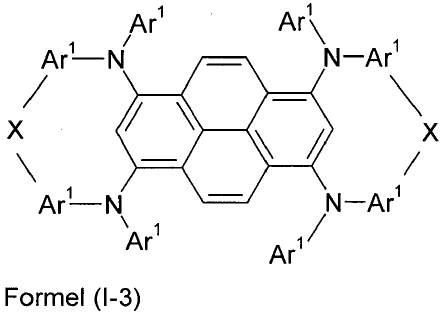 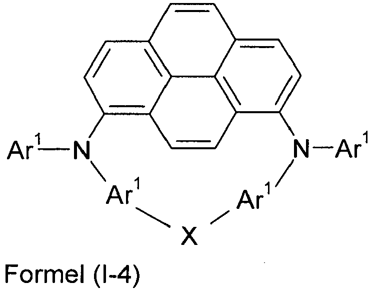 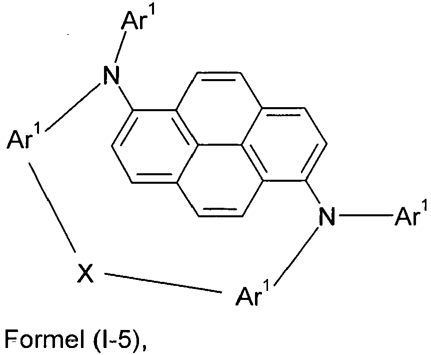 wobei die Pyrengruppe an jeder freien Position mit einem Rest R¹ substituiert sein kann, und wobei die Gruppen definiert sind gemäß einem oder mehreren der Ansprüche 1 bis 10.

12. Oligomer, Polymer oder Dendrimer enthaltend eine oder mehrere Verbindungen nach einem oder mehreren der Ansprüche 1 bis 11, wobei die Bindung(en) zum Polymer, Oligomer oder Dendrimer an beliebigen, in Formel (I) mit R¹, R² oder R³ substituierten Positionen lokalisiert sein können.

13. Formulierung, enthaltend mindestens eine Verbindung nach einem oder mehreren der Ansprüche 1 bis 11 sowie mindestens ein Lösungsmittel.

14. Elektronische Vorrichtung, gewählt aus organischen integrierten Schaltungen, organischen Feld-Effekt-Transistoren, organischen Dünnfilmtransistoren, organischen lichtemittierenden Transistoren, organischen Solarzellen, organischen optischen Detektoren, organischen Photorezeptoren, organischen Feld-Quench-Devices, organischen lichtemittierenden elektrochemischen Zellen, organischen Laserdioden und organischen Elektrolumineszenzvorrichtungen, enthaltend mindestens eine Verbindung nach einem oder mehreren der Ansprüche 1 bis 11.

15. Elektronische Vorrichtung nach Anspruch 14, gewählt aus organischen Elektrolumineszenzvorrichtungen, **dadurch gekennzeichnet, dass** die Verbindung nach einem oder mehreren der Ansprüche 1 bis 11 als emittierende Verbindung in einer emittierenden Schicht vorliegt.

16. Verfahren zur Herstellung einer Verbindung nach einem oder mehreren der Ansprüche 1 bis 11, **dadurch gekennzeichnet, dass** es mindestens eine übergangsmetallkatalysierte Kupplungsreaktion umfasst.

17. Verwendung einer Verbindung nach einem oder mehreren der Ansprüche 1 bis 11 in einer elektronischen Vorrichtung.

## Claims

1. Compound of a formula (I) 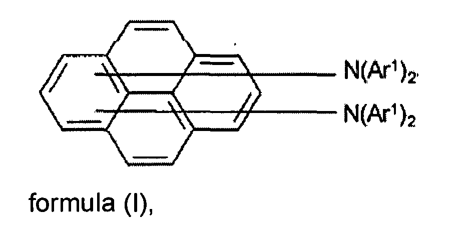 where the pyrene group may be substituted at each free position by a radical R¹, and where:
in the formula (I), at least one group Ar¹ which is a constituent of a group N(Ar¹)₂ is linked via a group X to another group Ar¹ that is a constituent of another group N(Ar¹)₂;
Ar¹ is on each occurrence, identically or differently, an aromatic or heteroaromatic ring system having 6 to 30 aromatic ring atoms, which may be substituted by one or more radicals R³, where groups Ar¹ may be linked here via groups X;
X is on each occurrence, identically or differently, a single bond, or a divalent group selected from an aryl or heteroaryl group having 6 to 20 aromatic ring atoms, which may be substituted by one or more radicals R², or is BR², C(R²)₂, -R²C=CR²-, C(=O), Si(R²)₂, NR², PR², P(=O)R², O, S, S(=O), S(=O)₂, or a combination of 2, 3, 4 or 5 identical or different divalent groups of those mentioned;
R¹ is on each occurrence, identically or differently, H, D, F, C(=O)R⁴, CN, Si(R⁴)₃, N(Ar¹)2, N(R⁴)₂, P(=O)(R⁴)₂, OAr¹, S(=O)R⁴, S(=O)₂R⁴, a straight-chain alkyl or alkoxy group having 1 to 20 C atoms or a branched or cyclic alkyl or alkoxy group having 3 to 20 C atoms or an alkenyl or alkynyl group having 2 to 20 C atoms, where the above-mentioned groups may each be substituted by one or more radicals R⁴ and where one or more CH₂ groups in the above-mentioned groups may be replaced by -R⁴C=CR⁴-, -C=C-, Si(R⁴)₂, C=O, C=NR⁴, -C(=O)O-, -C(=O)NR⁴-, NR⁴, P(=O)(R⁴), -O-, -S-, SO or SO₂, or an aromatic or heteroaromatic ring system having 5 to 30 aromatic ring atoms, which may in each case be substituted by one or more radicals R⁴, where two or more radicals R¹ may be linked to one another and may form a ring;
R² is on each occurrence, identically or differently, H, D, F, C(=O)R⁴, CN, Si(R⁴)₃, N(R⁴)₂, P(=O)(R⁴)₂, S(=O)R⁴, S(=O)₂R⁴, a straight-chain alkyl or alkoxy group having 1 to 20 C atoms or a branched or cyclic alkyl or alkoxy group having 3 to 20 C atoms or an alkenyl or alkynyl group having 2 to 20 C atoms, where the above-mentioned groups may each be substituted by one or more radicals R⁴ and where one or more CH₂ groups in the above-mentioned groups may be replaced by -R⁴C=CR⁴-, -C=C-, Si(R⁴)₂, C=O, C=NR⁴, -C(=O)O-, -C(=O)NR⁴-, NR⁴, P(=O)(R⁴), -O-, -S-, SO or SO₂, or an aromatic or heteroaromatic ring system having 5 to 30 aromatic ring atoms, which may in each case be substituted by one or more radicals R⁴, where two or more radicals R² may be linked to one another and may form a ring;
R³ is on each occurrence, identically or differently, H, D, F, C(=O)R⁴, CN, Si(R⁴)₃, N(R⁴)₂, P(=O)(R⁴)₂, S(=O)R⁴, S(=O)₂R⁴, a straight-chain alkyl or alkoxy group having 1 to 20 C atoms or a branched or cyclic alkyl or alkoxy group having 3 to 20 C atoms or an alkenyl or alkynyl group having 2 to 20 C atoms, where the above-mentioned groups may each be substituted by one or more radicals R⁴ and where one or more CH₂ groups in the above-mentioned groups may be re-placed by -R⁴C=CR⁴-, -C≡C-, Si(R⁴)₂, C≡O, C≡NR⁴, -C(=O)O-, -C(=O)NR⁴-, NR⁴, P(=O)(R⁴), -O-, -S-, SO or SO₂, or an aromatic or heteroaromatic ring system having 5 to 30 aromatic ring atoms, which may in each case be substituted by one or more radicals R⁴, where two or more radicals R³ may be linked to one another and may form a ring;
R⁴ is on each occurrence, identically or differently, H, D, F or an aliphatic, aromatic or heteroaromatic organic radical having 1 to 20 C atoms, in which, in addition, one or more H atoms may be replaced by D or F; two or more substituents R⁴ here may be linked to one another and may form a ring.

2. Compound according to Claim 1, **characterised in that** the group X is on each occurrence, identically or differently, a divalent group selected from C(R²)₂, NR², O and S, even more preferably a divalent group selected from C(R²)₂ and NR².

3. Compound according to Claim 1 or 2, **characterised in that** the compound of the formula (I) contains precisely one or precisely 2 groups X, so that precisely 1 pair of groups Ar¹ or precisely 2 pairs of groups Ar¹ are linked to one another via in each case a group X.

4. Compound according to one or more of Claims 1 to 3, **characterised in that** Ar¹ is on each occurrence, identically or differently, an aromatic ring system having 6 to 12 aromatic ring atoms, which may be substituted by one or more radicals R³, and is preferably phenyl, which may be substituted by one or more radicals R³.

5. Compound according to Claim 4, **characterised in that** Ar¹ is a phenyl group, which may be substituted by one or more radicals R³, and **in that** the bond to the group X and to the nitrogen atom on the phenyl group are in the meta position to one another.

6. Compound according to one or more of Claims 1 to 5, **characterised in that** the pyrene group is substituted by at least one radical R¹ which is not equal to H.

7. Compound according to one or more of Claims 1 to 6, **characterised in that** the pyrene group is substituted by at least one radical R¹ which is selected from a straight-chain alkyl group having 1 to 8 C atoms or a branched or cyclic alkyl group having 3 to 8 C atoms, where the alkyl groups may be substituted by one or more radicals R⁴, or from an aryl or heteroaryl group having 6 to 14 aromatic ring atoms, which may be substituted by one or more radicals R⁴.

8. Compound according to one or more of Claims 1 to 7, **characterised in that** radicals R² on a group X which represents C(R²)₂ or Si(R²)₂ are selected from a straight-chain alkyl group having 1 to 8 C atoms or a branched or cyclic alkyl group having 3 to 8 C atoms, where the alkyl groups may be substituted by one or more radicals R⁴, or from an aryl or heteroaryl group having 6 to 14 aromatic ring atoms, which may be substituted by one or more radicals R⁴.

9. Compound according to one or more of Claims 1 to 8, **characterised in that** the groups N(Ar¹)₂ in formula (I) are bonded at positions selected from positions 1, 3, 6 and 8 of the pyrene.

10. Compound according to one or more of Claims 1 to 9, **characterised in that** groups N(Ar¹)₂ that are connected to one another via groups X are bonded at positions 1 and 3 of the pyrene.

11. Compound according to one or more of Claims 1 to 10, **characterised in that** it conforms to one of the formulae (I-1) to (I-5): 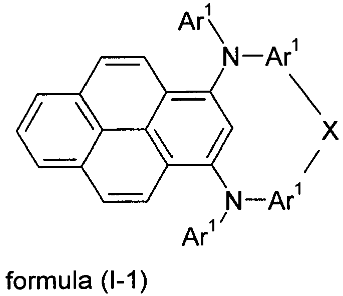 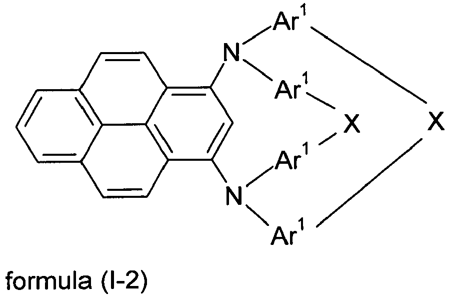 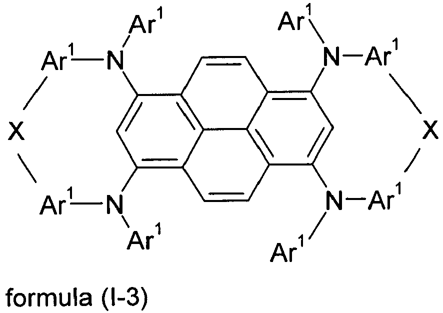 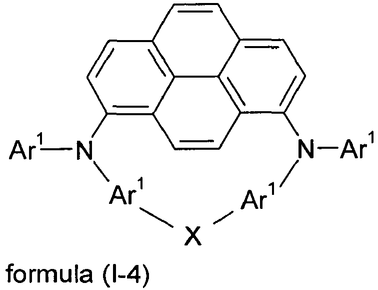 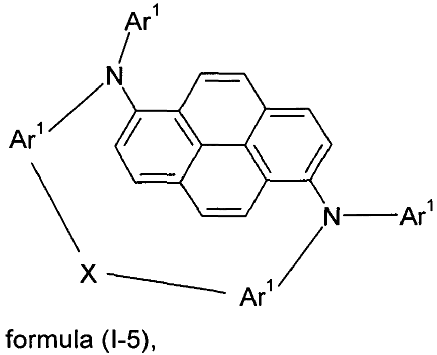 where the pyrene group may be substituted at each free position by a radical R¹, and where the groups are defined in accordance with one or more of Claims 1 to 10.

12. Oligomer, polymer or dendrimer containing one or more compounds according to one or more of Claims 1 to 11, where the bond(s) to the polymer, oligomer or dendrimer may be localised at any desired positions in formula (I) that arensubstituted by R¹, R² or R³.

13. Formulation comprising at least one compound according to one or more of Claims 1 to 11 and at least one solvent.

14. Electronic device, selected from organic integrated circuits, organic field-effect transistors, organic thin-film transistors, organic light-emitting transistors, organic solar cells, organic optical detectors, organic photoreceptors, organic field-quench devices, organic light-emitting electrochemical cells, organic laser diodes and organic electroluminescent devices, comprising at least one compound according to one or more of Claims 1 to 11.

15. Electronic device according to Claim 14, selected from organic electroluminescent devices, **characterised in that** the compound according to one or more of Claims 1 to 11 is present as emitting compound in an emitting layer.

16. Process for the preparation of a compound according to one or more of Claims 1 to 11, **characterised in that** it includes at least one transition metal-catalysed coupling reaction.

17. Use of a compound according to one or more of Claims 1 to 11 in an electronic device.

## Revendications

1. Composé de la formule (I) : 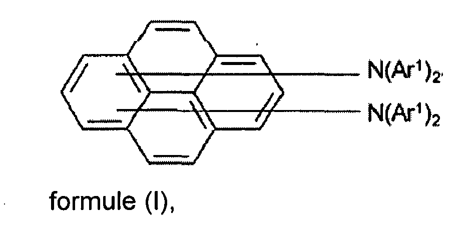 dans laquelle le groupe pyrène peut être substitué au niveau de chaque position libre par un radical R¹, et où :
dans la formule (1), au moins un groupe Ar¹ qui est un constituant d'un groupe N(Ar¹)₂ est lié via un groupe X à un autre groupe Ar¹ qui est un constituant d'un autre groupe N(Ar¹)₂ ;
Ar¹ est pour chaque occurrence, de manière identique ou différente, un système de cycle aromatique ou hétéroaromatique comportant 6 à 30 atomes de cycle aromatique, lequel peut être substitué par un radical ou plusieurs radicaux R³, où des groupes Ar¹ peuvent être liés ici via des groupes X ;
X est pour chaque occurrence, de manière identique ou différente, une liaison simple, ou un groupe divalent choisi parmi un groupe aryle ou hétéroaryle comportant 6 à 20 atomes de cycle aromatique, lequel peut être substitué par un radical ou plusieurs radicaux R², ou est BR², C(R²)₂, -R²C=CR²-, C(=O), Si(R²)₂, NR², PR², P(=O)R², O, S, S(=O), S(=O)₂, ou une combinaison de 2, 3, 4 ou 5 groupes divalents identiques ou différents pris parmi ceux mentionnés ;
R¹ est pour chaque occurrence, de manière identique ou différente, H, D, F, C(=O)R⁴ CN, Si(R⁴)₃, N(Ar¹)₂, N(R⁴)₂, P(=O)(R⁴)₂, OAr¹, S(=O)R⁴ S(=O)₂R⁴, un groupe alkyle ou alcoxy en chaîne droite comportant 1 à 20 atome(s) de C ou un groupe alkyle ou alcoxy ramifié ou cyclique comportant 3 à 20 atomes de C ou un groupe alkényle ou alkynyle comportant 2 à 20 atomes de C, où les groupes mentionnés ci avant peuvent chacun être substitués par un radical ou plusieurs radicaux R⁴ et où un ou plusieurs groupe(s) CH₂ dans les groupes mentionnés ci avant peut/peuvent être remplacé(s) par -R⁴C=CR⁴-, -C≡C-, Si(R⁴)₂, C=O, C=NR⁴, -C(=O)O-, -C(=O)NR⁴-, NR⁴, P(=O)(R⁴), -O-, -S-, SO ou SO₂, ou un système de cycle aromatique ou hétéroaromatique comportant 5 à 30 atomes de cycle aromatique, lequel peut dans chaque cas être substitué par un radical ou plusieurs radicaux R⁴, où deux radicaux R¹ ou plus peuvent être liés l'un à l'autre ou les uns aux autres et peuvent former un cycle ;
R² est pour chaque occurrence, de manière identique ou différente, H, D, F, C(=O)R⁴ CN, Si(R⁴)₃, N(R⁴)₂, P(=O)(R⁴)₂, S(=O)R⁴ S(=O)₂R⁴ un groupe alkyle ou alcoxy en chaîne droite comportant 1 à 20 atome(s) de C ou un groupe alkyle ou alcoxy ramifié ou cyclique comportant 3 à 20 atomes de C ou un groupe alkényle ou alkynyle comportant 2 à 20 atomes de C, où les groupes mentionnés ci avant peuvent chacun être substitués par un radical ou plusieurs radicaux R⁴ et où un ou plusieurs groupe(s) CH₂ dans les groupes mentionnés ci avant peut/peuvent être remplacé(s) par -R⁴C=CR⁴-, -C=C-, Si(R⁴)₂, C=O, C=NR⁴, -C(=O)O-, -C(=O)NR⁴-, NR⁴, P(=O)(R⁴), -O-, -S-, SO ou SO₂, ou un système de cycle aromatique ou hétéroaromatique comportant 5 à 30 atomes de cycle aromatique, lequel peut dans chaque cas être substitué par un radical ou plusieurs radicaux R⁴, où deux radicaux R² ou plus peuvent être liés l'un à l'autre ou les uns aux autres et peuvent former un cycle ;
R³ est pour chaque occurrence, de manière identique ou différente, H, D, F, C(=O)R⁴, CN, Si(R⁴)₃, N(R⁴)₂, P(=O)(R⁴)₂, S(=O)R⁴, S(=O)₂R⁴, un groupe alkyle ou alcoxy en chaîne droite comportant 1 à 20 atome(s) de C ou un groupe alkyle ou alcoxy ramifié ou cyclique comportant 3 à 20 atomes de C ou un groupe alkényle ou alkynyle comportant 2 à 20 atomes de C, où les groupes mentionnés ci avant peuvent chacun être substitués par un radical ou plusieurs radicaux R⁴ et où un ou plusieurs groupe(s) CH₂ dans les groupes mentionnés ci avant peut/peuvent être remplacé(s) par -R⁴C=CR⁴-, -C≡C-, Si(R⁴)₂, C=O, C=NR⁴, -C(=O)O-, -C(=O)NR⁴-, NR⁴, P(=O)(R⁴), -O-, -S-, SO ou SO₂, ou un système de cycle aromatique ou hétéroaromatique comportant 5 à 30 atomes de cycle aromatique, lequel peut dans chaque cas être substitué par un radical ou plusieurs radicaux R⁴, où deux radicaux R³ ou plus peuvent être liés l'un à l'autre ou les uns aux autres et peuvent former un cycle ;
R⁴ est pour chaque occurrence, de manière identique ou différente, H, D, F ou un radical organique aliphatique, aromatique ou hétéroaromatique comportant 1 à 20 atome(s) de C, où, en outre, un ou plusieurs atome(s) de H peut/peuvent être remplacé(s) par D ou F ; deux substituants R⁴ ou plus peuvent être ici liés l'un à l'autre ou les uns aux autres et peuvent former un cycle.

2. Composé selon la revendication 1, **caractérisé en ce que** le groupe X est pour chaque occurrence, de manière identique ou différente, un groupe divalent choisi parmi C(R²)₂, NR², O et S, de façon encore davantage préférable, un groupe divalent choisi parmi C(R²)₂ et NR².

3. Composé selon la revendication 1 ou 2, **caractérisé en ce que** le composé de la formule (1) contient précisément un seul groupe ou précisément 2 groupes X, de telle sorte que les groupes de précisément 1 paire de groupes Ar¹ ou les groupes de précisément 2 paires de groupes Ar¹ soient lié(s) l'un à l'autre ou les uns aux autres via dans chaque cas un groupe X.

4. Composé selon une ou plusieurs des revendications 1 à 3, **caractérisé en ce que** Ar¹ est pour chaque occurrence, de manière identique ou différente, un système de cycle aromatique comportant 6 à 12 atomes de cycle aromatique, lequel peut être substitué par un radical ou plusieurs radicaux R³, et est de façon préférable phényle, lequel peut être substitué par un radical ou plusieurs radicaux R³.

5. Composé selon la revendication 4, **caractérisé en ce que** Ar¹ est un groupe phényle, lequel peut être substitué par un radical ou plusieurs radicaux R³, et **en ce que** la liaison sur le groupe X et la liaison sur l'atome d'azote sur le groupe phényle sont à la position méta l'une par rapport à l'autre.

6. Composé selon une ou plusieurs des revendications 1 à 5, **caractérisé en ce que** le groupe pyrène est substitué par au moins un radical R¹ qui n'est pas égal à H.

7. Composé selon une ou plusieurs des revendications 1 à 6, **caractérisé en ce que** le groupe pyrène est substitué par au moins un radical R¹ qui est choisi parmi un groupe alkyle en chaîne droite comportant 1 à 8 atome(s) de C ou un groupe alkyle ramifié ou cyclique comportant 3 à 8 atomes de C, où les groupes alkyle peuvent être substitués par un radical ou plusieurs radicaux R⁴, ou parmi un groupe aryle ou hétéroaryle comportant 6 à 14 atomes de cycle aromatique, lequel peut être substitué par un radical ou plusieurs radicaux R⁴.

8. Composé selon une ou plusieurs des revendications 1 à 7, **caractérisé en ce que** des radicaux R² sur un groupe X qui représente C(R²)₂ ou Si(R²)₂ sont choisis parmi un groupe alkyle en chaîne droite comportant 1 à 8 atome(s) de C ou un groupe alkyle ramifié ou cyclique comportant 3 à 8 atomes de C, où les groupes alkyle peuvent être substitués par un radical ou plusieurs radicaux R⁴, ou parmi un groupe aryle ou hétéroaryle comportant 6 à 14 atomes de cycle aromatique, lequel peut être substitué par un radical ou plusieurs radicaux R⁴.

9. Composé selon une ou plusieurs des revendications 1 à 8, **caractérisé en ce que** les groupes N(Ar**¹**)**₂** dans la formule (I) sont liés au niveau de positions choisies parmi les positions 1, 3, 6 et 8 du pyrène.

10. Composé selon une ou plusieurs des revendications 1 à 9, **caractérisé en ce que** des groupes N(Ar**¹**)**₂** qui sont connectés les uns aux autres via des groupes X sont liés au niveau de positions 1 et 3 du pyrène.

11. Composé selon une ou plusieurs des revendications 1 à 10, **caractérisé en ce qu'**il est conforme à l'une des formules (I-1) à (I-5) : 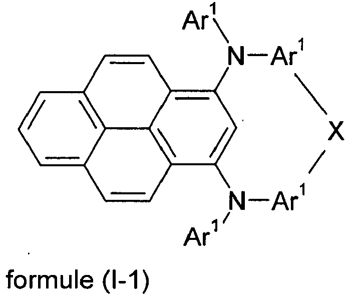 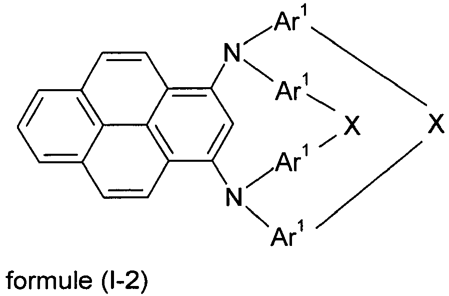 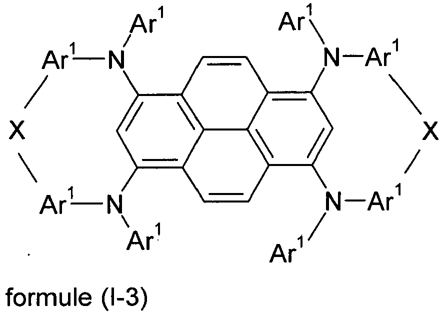 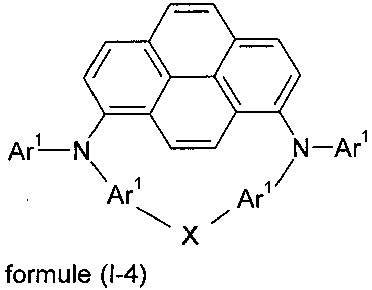 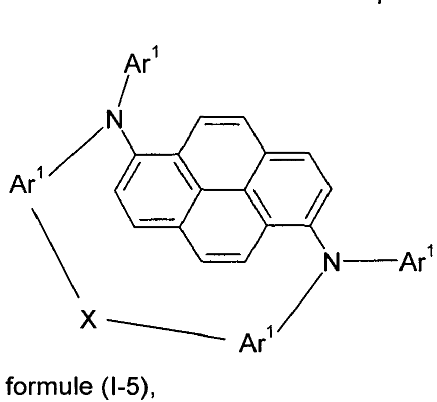 dans lesquelles le groupe pyrène peut être substitué au niveau de chaque position libre par un radical R¹, et où les groupes sont définis selon une ou plusieurs des revendications 1 à 10.

12. Oligomère, polymère ou dendrimère contenant un ou plusieurs composé(s) selon une ou plusieurs des revendications 1 à 11, où la liaison/les liaisons sur le polymère, l'oligomère ou le dendrimère peut/ peuvent être localisée(s) au niveau de n'importe quelles positions souhaitées dans la formule (I) qui sont substituées par R¹, R² ou R³.

13. Formulation comprenant au moins un composé selon une ou plusieurs des revendications 1 à 11 et au moins un solvant.

14. Dispositif électronique, choisi parmi les circuits intégrés organiques, les transistors à effet de champ organiques, les transistors à film mince organiques, les transistors à émission de lumière organiques, les cellules solaires organiques, les détecteurs optiques organiques, les photorécepteurs organiques, les dispositifs à extinction de champ organiques, les cellules électrochimiques à émission de lumière organiques, les diodes laser organiques et les dispositifs électroluminescents organiques, comprenant au moins un composé selon une ou plusieurs des revendications 1 à 11.

15. Dispositif électronique selon la revendication 14, choisi parmi les dispositifs électroluminescents organiques, **caractérisé en ce que** le composé selon une ou plusieurs des revendications 1 à 11 est présent en tant que composé d'émission dans une couche d'émission.

16. Procédé pour la préparation d'un composé selon une ou plusieurs des revendications 1 à 11, **caractérisé en ce qu'**il inclut au moins une réaction de couplage catalysée par métal de transition.

17. Utilisation d'un composé selon une ou plusieurs des revendications 1 à 11 dans un dispositif électronique.
